# FASCICULE DE BREVET EUROPEEN

(11) **EP 2 046 428 B1**
(45) Date de publication et mention de la délivrance du brevet: **26.03.2014**
(21) Numéro de dépôt: 07823590.0
(22) Date de dépôt: 17.07.2007
(51) Int. Cl.: A61M 15/00

(54) **DISPOSITIF DE DISTRIBUTION DE PRODUIT FLUIDE**
SPENDER FÜR EIN FLÜSSIGPRODUKT
FLUID-PRODUCT DISPENSING DEVICE

(30) Priorité: 25.07.2006 FR 0653101
(43) Date de publication de la demande: 15.04.2009
(73) Titulaire: Aptar France SAS, 27110 Le Neubourg (FR)
(72) Inventeur: POCOCK, Andrew Gordon, Ickleton Cambridgeshire CB10 1SX (GB); KAY, Stuart Brian William, Ickleton Cambridgeshire CB10 1SX (GB); GREENHALGH, Paul, Ickleton Cambridgeshire CB10 1SX (GB); O'HARA, Wayne, Ickleton Cambridgeshire CB10 1SX (GB); DONNETTE, Xavier, F-78370 Plaisir (FR)
(74) Mandataire: CAPRI
(86) Numéro de dépôt international: PCT/FR2007/051670
(87) Numéro de publication internationale: WO 2008/012456

(56) Documents cités:
- WO-A-2006/071512
- US-A- 6 012 454
- US-A1- 2005 081 851
- US-B1- 6 880 555

## Description

La présente invention concerne un dispositif de distribution de produit fluide, et plus particulièrement un inhalateur de poudre sèche.

Les inhalateurs de poudre sèche sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blister allongé ou des blisters disposés sur un disque circulaire rotatif. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre évidemment, l'utilisation est plus complexe, puisque l'utilisateur est obligé de charger une capsule dans l'inhalateur avant chaque utilisation. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. En variante, il a été proposé de percer la paroi ou couche de fermeture. Ceci présente l'inconvénient que les parties de paroi découpées risquent de retenir une partie de la dose à l'intérieur du réservoir, la précision et reproductibilité de dosage n'étant donc pas garanties. Les documents WO 2006/071512, US-6 880 555, US-6 012 454 et US 2005/081851 décrivent des dispositifs de l'art antérieur.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un dispositif qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un dispositif qui évite tout risque de sous-dosage, l'ouverture du réservoir, l'expulsion de la dose et le comptage de la dose émise ne se produisant qu'après l'inhalation de l'utilisateur. De plus, la présente invention a pour but d'éviter tout risque de perte de dose en l'absence d'inhalation même si l'utilisateur manipule le dispositif.

La présente invention a encore pour but de fournir un dispositif permettant de compter le nombre de doses émises ou restant à émettre.

La présente invention a donc pour objet un dispositif de distribution de produit fluide tel que décrit dans la revendication 1.

La présente invention a aussi pour objet un dispositif de distribution de produit fluide tel que décrit dans la revendication 2.

Des variantes avantageuses sont décrites dans les revendications dépendantes.

Ces caractéristiques et avantages et d'autres de la présente invention apparaîtront plus clairement au cours de la description détaillée suivante de plusieurs modes et variantes de réalisation de celle-ci, faite en référence aux dessins joints, donnés à titres d'exemples non limitatifs, sur lesquels,
- la figure 1 est une vue schématique externe d'un dispositif selon une variante avantageuse de l'invention,
- la figure 2 est une vue schématique en section d'un dispositif selon un mode de réalisation de l'invention, en position de fermeture,
- la figure 3 est une vue similaire à la figure 2, en cours d'ouverture,
- la figure 4 est une vue similaire à la figure 3, en position d'ouverture,
- la figure 5 est une vue similaire à la figure 4, après inhalation,
- la figure 6 est une vue similaire à la figure 5, en début de fermeture après inhalation,
- la figure 7 est une vue similaire à la figure 6, en cours de fermeture après inhalation,
- la figure 8 est une vue similaire à la figure 7, en fin de fermeture après inhalation,
- la figure 9 est une vue similaire à la figure 4, en début de fermeture en l'absence d'inhalation,
- la figure 10 est une vue schématique en section d'un dispositif selon un autre mode de réalisation de l'invention, en position de fermeture,
- la figure 11 est une vue similaire à la figure 10, en position d'ouverture,
- la figure 12 est une vue similaire à la figure 11, après inhalation,
- la figure 13 est une vue similaire à la figure 12, en cours de fermeture après inhalation,
- la figure 14 est une vue similaire à la figure 13, en fin de fermeture après inhalation,
- la figure 15 est une vue schématique d'un dispositif selon une variante de réalisation de l'invention, montrant en transparence le dispositif d'inhalation de doses, en position de fermeture,
- la figure 16 est une vue schématique en section du dispositif de la figure 15,
- la figure 17 est une vue en détail de la figure 16,
- la figure 18 est une vue similaire à la figure 15, en position d'ouverture,
- la figure 19 est une vue similaire à la figure 18, après inhalation,
- la figure 20 est une vue similaire à la figure 19, en fin de fermeture après inhalation,
- la figure 21 est une vue schématique en section d'un dispositif selon un autre mode de réalisation de l'invention, en position de fermeture,
- la figure 22 est une vue similaire à la figure 21, en cours d'ouverture,
- la figure 23 est une vue similaire à la figure 22, en position d'ouverture,
- la figure 24 est une vue similaire à la figure 23, après inhalation,
- la figure 25 est une vue similaire à la figure 24, en cours de fermeture après inhalation,
- la figure 26 est une vue similaire à la figure 25, en fin de fermeture après inhalation,
- les figures 27a et 27b sont des vues schématiques en section, respectivement de devant et de derrière, d'un autre mode de réalisation de l'invention, en position de fermeture ;
- les figures 28a et 28b sont des vues similaires aux figures 27a et 27b, en position d'ouverture,
- les figures 29a et 29b sont des vues similaires aux figures 28a et 28b, après inhalation,
- les figures 30a et 30b sont des vues similaires aux figures 29a et 29b, en cours de fermeture après inhalation,
- les figures 31a et 31b sont des vues similaires aux figures 30a et 30b, en fin de fermeture après inhalation,
- la figure 32 est une vue similaire à la figure 5, montrant schématiquement le support de réservoirs et les moyens d'ouverture selon une variante avantageuse de l'invention

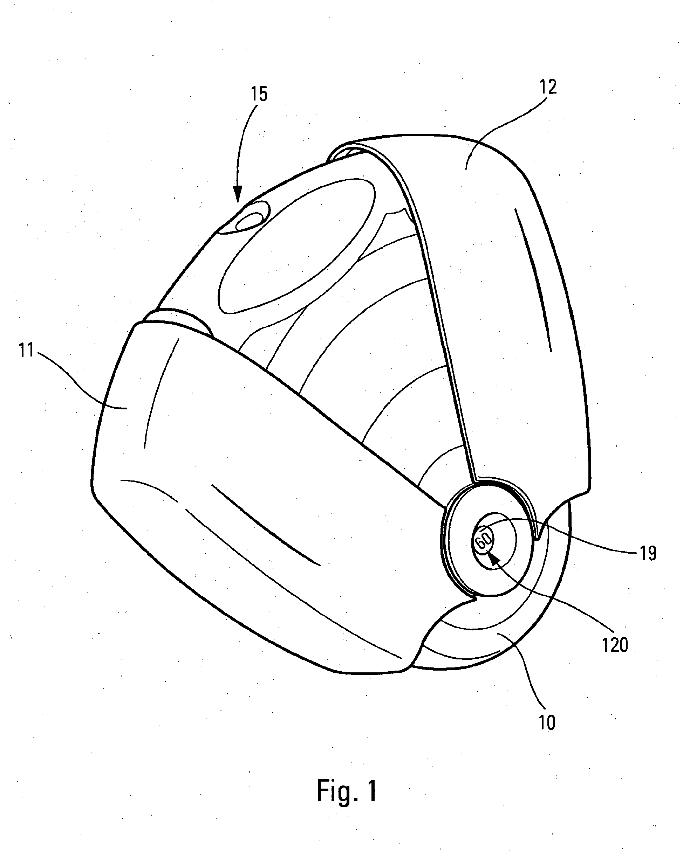

La figure 1 représente une vue externe d'un mode de réalisation d'un inhalateur de poudre sèche. Cet inhalateur comporte un corps central 10 sur lequel sont montées coulissantes deux éléments ou ailes latérales 11, 12 formant capot lorsque le dispositif est fermé et adaptées à être séparées l'une de l'autre pour ouvrir le dispositif et ainsi charger le dispositif comme cela sera décrit ci-après. Le corps 10 peut être de forme environ arrondie en partie basse, et relativement plat en partie haute, comme représenté sur la figure 1, mais il pourrait avoir tout autre forme appropriée. Le corps 10 comporte un orifice de distribution et d'inhalation 15 à travers lequel l'utilisateur va inhaler lors de l'actionnement du dispositif. Les deux parties latérales 11, 12 formant capot peuvent s'ouvrir par pivotement autour d'un axe de rotation commun comme représenté sur la figure 1, mais tout autre moyen d'ouverture du dispositif est envisageable. Par ailleurs, on pourrait ne prévoir qu'un seul élément de capot mobile par rapport au corps au lieu des deux représentés sur la figure 1.

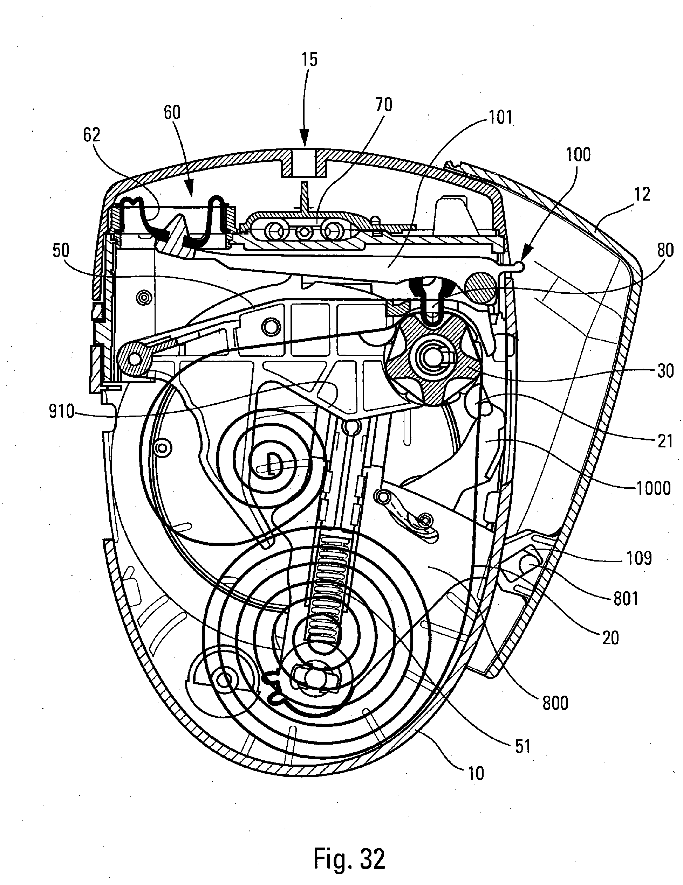

Avantageusement, le corps comporte une fenêtre 19 à travers laquelle le comptage des doses émises ou restant à émettre peut être affiché de manière visible pour l'utilisateur. Cette fenêtre 19 peut avantageusement être prévue sur ou proche de l'axe de rotation des éléments de capot 11, 12 formant le capot. A l'intérieur du corps, il peut être prévu un support 20 de réservoirs individuels 21. Les réservoirs sont avantageusement du type blisters, et le support de réservoir est de préférence une bande allongée, sur laquelle les blisters sont disposés les uns derrière les autres, de manière connue. Cette bande et ces blisters ne sont représentés que partiellement sur la figure 32 pour ne pas surcharger les autres dessins dans des buts de clarté. La bande de blister peut avantageusement être constituée d'une couche ou paroi de base formant des cavités recevant les doses de poudre, et d'une couche ou paroi de fermeture qui obture de manière étanche chacun desdits blisters. La bande de blister peut être enroulée à l'intérieur du corps et des moyens d'entraînement de bande 30 sont prévus pour progressivement dérouler cette bande de blister et amener un réservoir individuel ou blister respectif dans une position de distribution à chaque actionnement du dispositif. Lorsqu'un réservoir individuel a été vidé par une inhalation, la partie de bande comportant lesdits réservoirs vides est avantageusement adaptée à s'enrouler dans un autre endroit dudit corps 10.

Des moyens d'ouverture de réservoir 80 sont prévus dans ou solidaires du corps 10, ces moyens d'ouverture comportant des moyens de perçage et/ou de coupage de la couche de fermeture des blisters. Ces moyens d'ouverture ne sont également représentés que schématiquement sur la figure 32 pour ne pas surcharger les autres dessins dans des buts de clarté.

Des moyens de support mobiles 50 sont adaptés à supporter au moins le réservoir qui est destiné à être ouvert lors de la prochaine inhalation. Ces moyens de supports mobiles 50 sont adaptés à déplacer le réservoir à vider contre lesdits moyens d'ouverture du dispositif lors de l'actionnement. Avantageusement, ces moyens de supports mobiles 50 sont sollicités par un élément de chargement élastiquement déformable, tel qu'un ressort, une tige ou tout autre élément élastique équivalent, ledit élément de chargement pouvant être préchargé, notamment lors de l'ouverture du dispositif. Avantageusement, les moyens de support mobiles 50 sont déplaçables entre une première position (position de non-distribution) et une seconde position (position de distribution) qui est la position d'ouverture de réservoir.

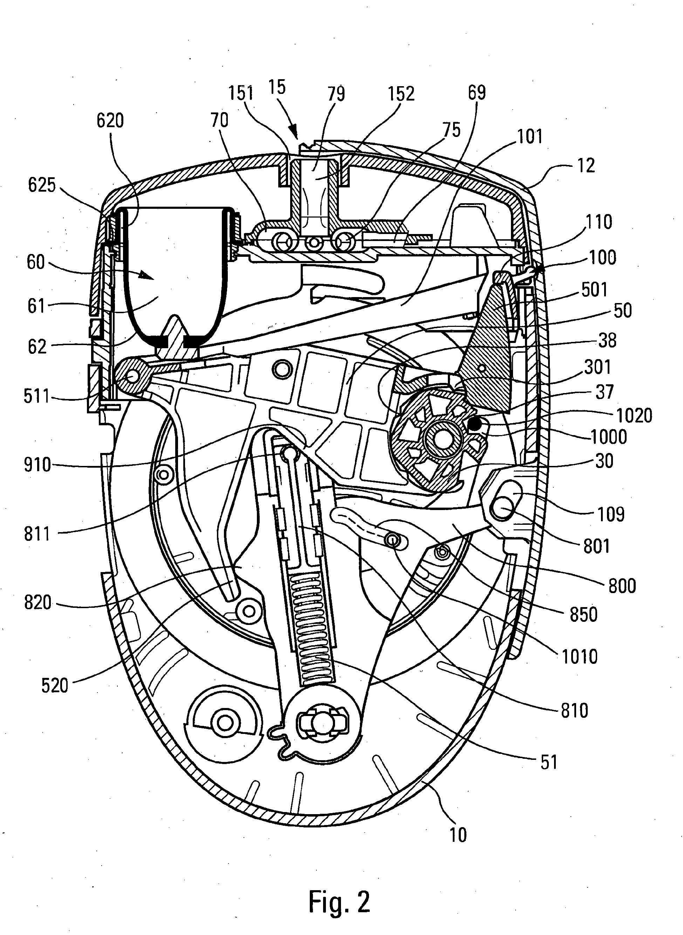

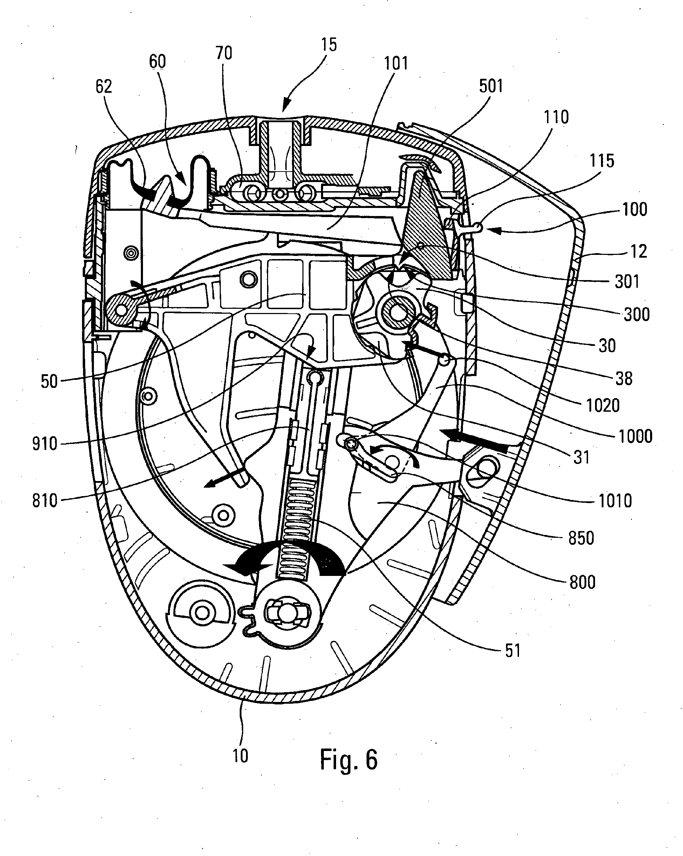

Avantageusement, les moyens de support mobiles 50 comportent une pièce sensiblement rigide, telle qu'une tige, articulée par rapport audit corps 10. Une roue de guidage 30 fixée de manière rotative sur lesdits moyens de support mobiles 50, reçoit et guide les blisters. Une rotation de cette roue de guidage 30 fait ainsi avancer la bande de blister dans une première direction. Dans une position angulaire particulière, un réservoir ou blister donné est toujours en position pour être ouvert par les moyens d'ouverture. Avantageusement, des moyens de positionnement en rotation 300 de ladite roue de guidage 30 peuvent être prévus pour déterminer précisément la position angulaire de ladite roue de guidage 30 après chaque rotation. Ces moyens de positionnement 300 peuvent, selon une variante avantageuse, comporter une projection ou doigt 301 dont une extrémité coopère élastiquement avec des encoches 38 prévues autour de ladite roue de guidage 30. Avantageusement, les encoches 38 comportent un profil environ en V qui guide automatiquement ledit doigt 301 vers la position centrale de l'encoche, assurant un positionnement angulaire précis à chaque rotation. Ces moyens de positionnement 300 sont notamment visibles sur les figures 2 et 6. La roue de guidage 30 forme de préférence les seuls moyens d'entraînement du support de réservoir. Eventuellement, une (ou plusieurs) roue complémentaire pourrait être prévue pour aider au guidage et/ou à l'entraînement du support de réservoirs.

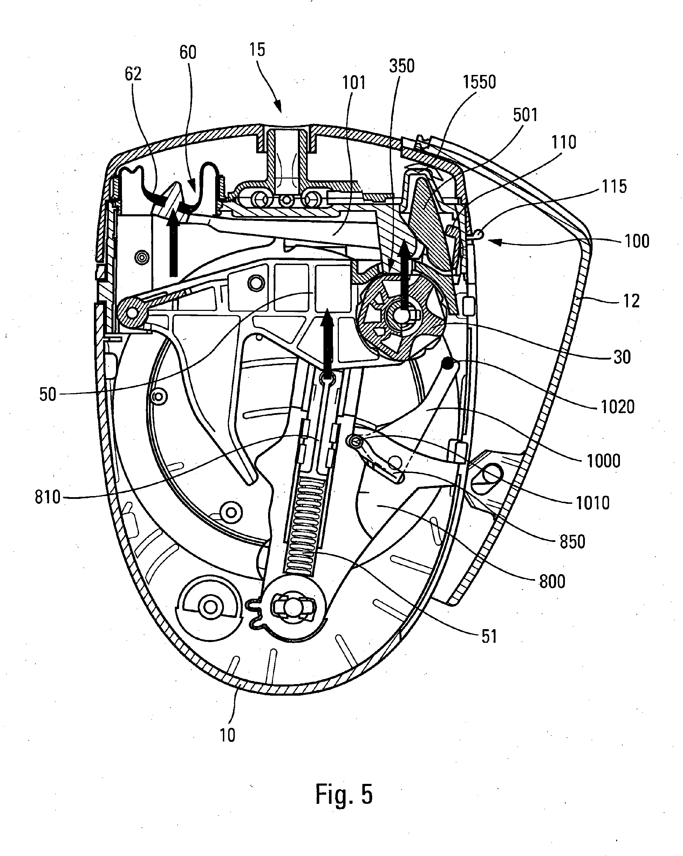

Avantageusement, des moyens de butée 350 sont prévus pour déterminer précisément la position de distribution de la roue de guidage 30 lors de chaque inhalation. Ces moyens de butée peuvent comporter un plot 350 adapté à coopérer en position de distribution avec une ou des surface(s) plane(s) correspondante(s) de la roue de guidage 30. De préférence, une surface plane est associée à chaque évidement. Dans cet exemple, cette butée 350 participe au positionnement correct en rotation de la roue de guidage 30 au moment où les moyens d'ouverture, notamment les moyens de perçage et/ou coupage, pénètrent dans le réservoir à vider. La butée 350 définit donc non seulement la profondeur de pénétration desdits moyens de perçage et/ou coupage dans le réservoir, mais aussi leur centrage par rapport au réservoir, afin de garantir un expulsion optimale de la poudre et une reproductibilité de dose élevée à chaque actionnement. Ces moyens de butée 350 peuvent être associés aux moyens de positionnement en rotation 300 susmentionnés de manière à prédéterminer de manière précise chaque position de la roue de guidage, en position de non-distribution, en position de distribution, et aussi lors des déplacements de la roue de guidage 30 entre ces positions. Ceci permet d'éviter des risques de blocage du dispositif en cas de mauvais positionnement de ladite roue de guidage. Ces moyens de butée 350 apparaissent notamment sur la figure 5.

Lorsque le réservoir se déplace vers sa position d'ouverture pour être ouvert par les moyens d'ouverture 80, ces moyens d'ouverture sont de préférence fixes par rapport au corps 10. Toutefois, il est envisageable que ces moyens de d'ouverture puissent également être mobiles pendant la phase d'ouverture du réservoir. Par exemple, les moyens d'ouverture pourraient se déplacer en direction du réservoir pendant que le réservoir se déplace en direction des moyens d'ouverture. Dans une autre variante, on pourrait également envisager que le réservoir et les moyens d'ouverture se déplacent dans la même direction lors de l'actionnement, le réservoir se déplaçant plus rapidement dans cette direction de sorte qu'il vient en contact avec lesdits moyens d'ouverture pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement des moyens 60 déplaçables et/ou déformables sous l'effet de l'inhalation, ces moyens 60 étant adaptés à libérer des moyens de blocage 100. Ces moyens 60 comprennent avantageusement une chambre d'air déformable 61 coopérant avec les moyens de blocage 100 desdits moyens de support mobiles 50. L'inhalation de l'utilisateur provoque la déformation de ladite chambre d'air déformable 61, permettant ainsi de libérer lesdits moyens de blocage 100 et donc de débloquer les moyens de support mobiles 50 pour permettre le déplacement de la roue de guidage 30, et donc du réservoir à vider, vers sa position d'ouverture. Avantageusement, cette chambre d'air 61 peut comprendre une membrane déformable 62, qui peut être reliée d'une part à l'orifice d'inhalation 15 et d'autre part auxdits moyens de blocage 100 de manière directe ou indirecte. Ainsi, lors de l'inhalation, la membrane 62 se déforme et/ou se contracte, provoquant ainsi le déplacement desdits moyens de blocage 100 dans une position de déblocage. Avantageusement, une poche ou diaphragme 62 peut former la chambre d'air 61. Cette poche 62 est reliée à l'orifice d'inhalation 15 via un canal 151 avantageusement disposé autour du canal d'expulsion 152 relié à une chambre de distribution 70. La poche 62 peut être fixée à une tige 101 reliée aux moyens de blocage 100, l'inhalation provoquant la déformation de la poche 62 et donc le pivotement de la tige 101 pour déplacer lesdits moyens de blocage 100. Avantageusement, la poche 62 peut être réalisée en silicone, et comporter un ourlet 620 adapté à former une étanchéité avec le corps 10. Pour ce faire, l'ourlet 620 peut se prolonger par une bride 625, également en silicone, qui va être comprimée par un partie d'encliquetage du corps 10 pour réaliser l'étanchéité, et notamment éviter toute perte de charge dans l'écoulement d'inhalation. En variante, la chambre d'air déformable pourrait être réalisée différemment, notamment par une membrane déformable quelconque.

L'inhalateur comporte en outre une chambre de distribution 70 qui est destinée à recevoir la dose de poudre après ouverture d'un réservoir respectif. Avantageusement, cette chambre de distribution 70 est pourvue d'au moins un élément sensiblement sphérique 75, tel qu'une bille, qui se déplace à l'intérieur de ladite chambre 70 pendant l'inhalation, pour améliorer la distribution du mélange air et poudre après ouverture d'un réservoir, afin d'augmenter l'efficacité du dispositif.

Selon une variante particulière, la chambre d'air déformable 61 coopère avec la chambre de distribution 70. Cette chambre de distribution 70 peut donc être reliée aux moyens d'ouverture du réservoir, et en particulier aux moyens de perçage et/ou de coupage, et comporte un orifice de distribution 79, avantageusement relié directement à l'orifice de distribution et d'inhalation 15 du dispositif. La membrane 62 peut donc être connectée d'une part à l'orifice d'inhalation 15, et d'autre part à cette chambre de distribution 70, dans le chemin d'écoulement d'inhalation de l'utilisateur. Il peut être avantageux que les moyens d'ouverture, en particulier les moyens de perçage et/ou de coupage soient formés directement sur ladite chambre de distribution 70, par exemple à l'extrémité d'un canal 69 menant à ladite chambre 70.

Après l'inhalation, lorsque l'utilisateur referme le dispositif, tous les composants reviennent vers leur position de repos initiale, à savoir que les moyens de support mobiles 50 pivotent par rapport à leur axe de pivotement en retour vers leur position de non distribution en éloignement des moyens d'ouverture de réservoir, et l'élément de chargement est également ramené dans sa position de repos initiale dans laquelle il n'est pas comprimé ou déformé. Le dispositif est alors prêt pour un nouveau cycle d'utilisation.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

Avantageusement, les moyens de support mobiles 50 qui supportent la roue de guidage 30 peuvent comporter une extension 501, indiquée sur les figures 2 et 5, qui sert notamment à coopérer avec les moyens de blocage 100. Cette extension peut en outre également servir à sensiblement boucher un trou 1550 prévu dans le dispositif, lorsque les moyens de support mobiles sont en position de distribution. Ainsi, en position de non distribution, l'écoulement d'inhalation passe en partie par le trou 1550. Après déplacement des moyens de support mobiles 50 vers la position de distribution, ce qui provoque sensiblement le bouchage du trou 1550, et l'ouverture du réservoir, l'écoulement d'inhalation est principalement canalisé vers ledit réservoir ouvert. Il s'en suit une meilleure efficacité lors de l'inhalation qui participe à assurer un vidage optimal du réservoir.

Selon un autre aspect avantageux de l'inhalateur, les réservoirs individuels ou blisters 21 sont formés sur une bande allongée 20, qui est stockée sous forme de bobine à l'intérieur du corps 10 du dispositif. Avantageusement, cette bande de blister enroulée est maintenue par des parois internes dudit corps 10 sans que son extrémité « arrière » (dans le sens de déplacement de la bande de blisters) ne soit fixée par rapport audit corps 10, ce qui permet un assemblage plus aisé de cette bande de blister à l'intérieur du dispositif. La bande de blister est déplacée avantageusement au moyen de la roue de guidage 30 qui présente avantageusement au moins un, de préférence plusieurs évidements 31, visibles sur la figure 6, dont la forme correspond sensiblement à celle des blisters. Ainsi, lorsque cette roue de guidage 30 tourne, elle entraîne la bande de blister dans la première direction. Bien entendu en variante ou de manière additionnelle, on pourrait utiliser d'autres moyens d'avancée de bande de blisters, par exemple en prévoyant un profil sur les bords longitudinaux latéraux de la bande de blister, ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous, à la manière d'une pellicule photo.

Selon encore un autre aspect de l'inhalateur, un dispositif de comptage ou d'indication de doses est également prévu. Ce dispositif peut comporter des chiffres ou symboles inscrits directement sur la bande de blister et visibles à travers une fenêtre appropriée 19 dans le corps 10 du dispositif. En variante, on pourrait envisager d'utiliser un ou plusieurs disque(s) rotatif(s) comportant les chiffres ou symboles, comme cela sera décrit ci-après.

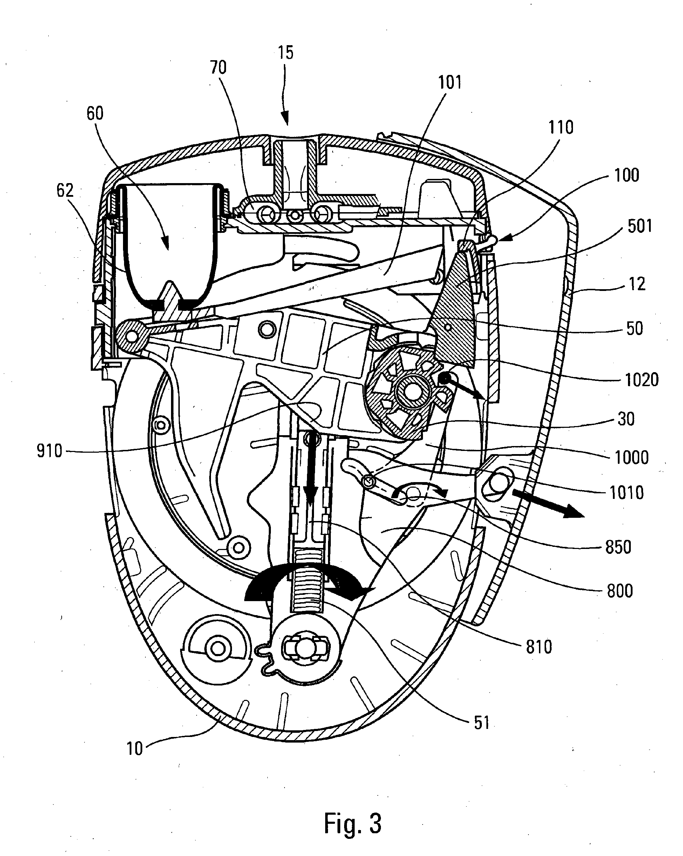

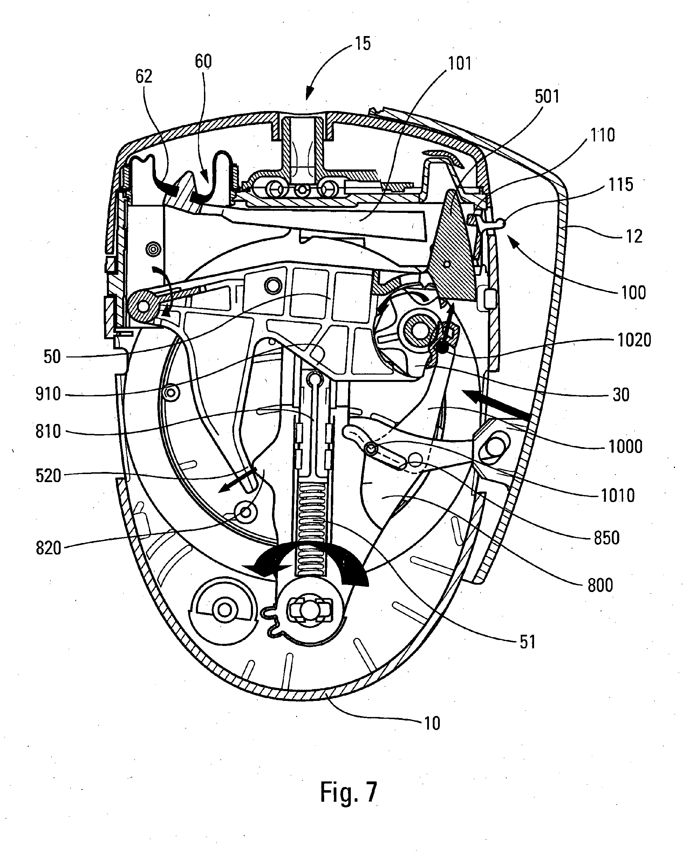

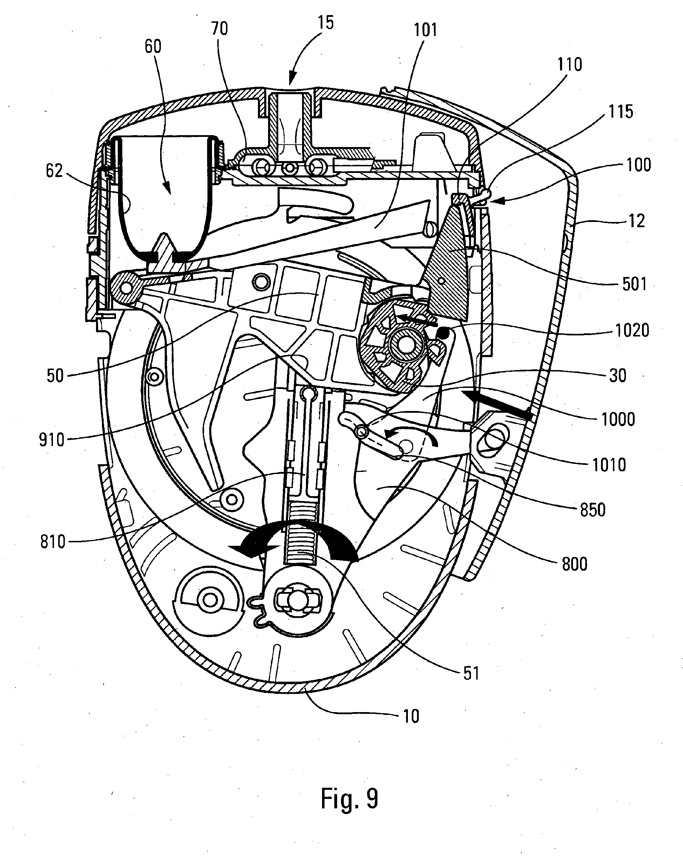

Les figures 2 à 9 représentent un mode de réalisation avantageux de la présente invention. En référence à ce mode de réalisation, le dispositif comporte un corps 10 sur lequel sont articulés deux éléments de capot 11, 12 autour avantageusement d'un axe d'articulation commun, comme cela est visible sur la figure 1. Pour des raisons de clarté, un seul des éléments de capot, en l'occurrence celui représenté sur le côté droit du dispositif et indiqué par la référence numérique 12, est représenté sur les figures. Les déplacements des différentes pièces sont indiqués schématiquement par des flèches sur certaines figures.

Ledit élément de capot mobile 12 est connecté à un organe d'armement 800, avantageusement par l'intermédiaire d'une ouverture 109 qui peut être de forme oblongue dans laquelle vient se placer un ergot ou similaire 801 dudit organe d'armement 800. Cet organe d'armement 800 est avantageusement monté pivotant sur le corps 10 autour d'un axe de pivotement. Cet organe d'armement 800 supporte l'élément de chargement 51 qui, dans ce mode de réalisation, est réalisé sous la forme d'un ressort à boudins. Ce ressort 51 coopère avec une tige 810, connectée d'un côté audit ressort à boudin 51 et de l'autre côté à une surface de came 910 prévue sur lesdits moyens de support mobiles 50, qui sera décrite ultérieurement. La tige 810, lorsque l'organe d'armement 800 est déplacé autour de son axe de pivotement lors du déplacement de l'élément de capot mobile 12, est donc adaptée à comprimer le ressort 51 lorsque l'élément de capot 12 est ouvert, et à décomprimer ledit ressort 51 lorsque ledit élément de capot 12 est refermé. Avantageusement, la tige 810 comporte, dans sa partie en contact avec la surface de came 910, une partie arrondie 811, telle qu'une extrémité en forme de boule, pour favoriser le glissement de la tige 810 sur ladite surface de came 910. L'organe d'armement 800 comporte en outre une projection 820 adaptée à coopérer avec une partie d'extension 520 desdits moyens de support mobiles 50, comme cela sera décrit plus en détail ci-après. L'organe d'armement 800 comporte en outre des moyens de guidage 850, avantageusement formés sous la forme d'une rainure, dans laquelle est reçu une projection 1010 solidaire d'un élément d'entraînement 1000, qui sera également décrit plus en détail ci-après. Avantageusement, ladite rainure 850 comporte au moins deux parties de pente différentes, dont la fonction sera également décrite ultérieurement.

Les moyens de support mobiles 50 sont dans ce mode de réalisation réalisés sous la forme d'une pièce montée pivotante sur le corps autour d'un axe de pivotement 511. Ces moyens de support mobiles 50 incorporent également une extension 501, avantageusement en forme d'aileron. La surface de came précitée 910 est formée sur lesdits moyens de support mobiles 50 de sorte que lorsque le ressort 51 est chargé lors de l'ouverture de l'élément de capot mobile 12, lesdits moyens de support mobiles 50 sont sollicités vers leur position de distribution par ladite tige 810 poussée par le ressort 51 comprimé. Des moyens de blocage 100 sont prévus pour retenir lesdits moyens de support mobiles 50 dans ladite position de non distribution, représentée notamment sur la figure 2. Lesdits moyens de blocage 100 comportent avantageusement un élément de blocage 110 adapté à coopérer avec ladite extension 501 desdits moyens de support mobiles 50. Lesdits moyens de blocage 100 sont avantageusement connectés au diaphragme déformable 62 sensible à l'inhalation de l'utilisateur par l'intermédiaire d'une tige 101, de sorte que lors de l'inhalation de l'utilisateur, ledit diaphragme se déforme, faisant ainsi pivoter la tige 101 et par la même ledit élément de blocage 110, libérant ainsi l'extension 501. Ceci permet le déplacement desdits moyens de support mobiles 50 vers leur position de distribution sous l'effet de la force exercée par le ressort comprimé 51. Ce déplacement des moyens de support mobiles 50 provoque l'ouverture d'un réservoir individuel comme décrit précédemment.

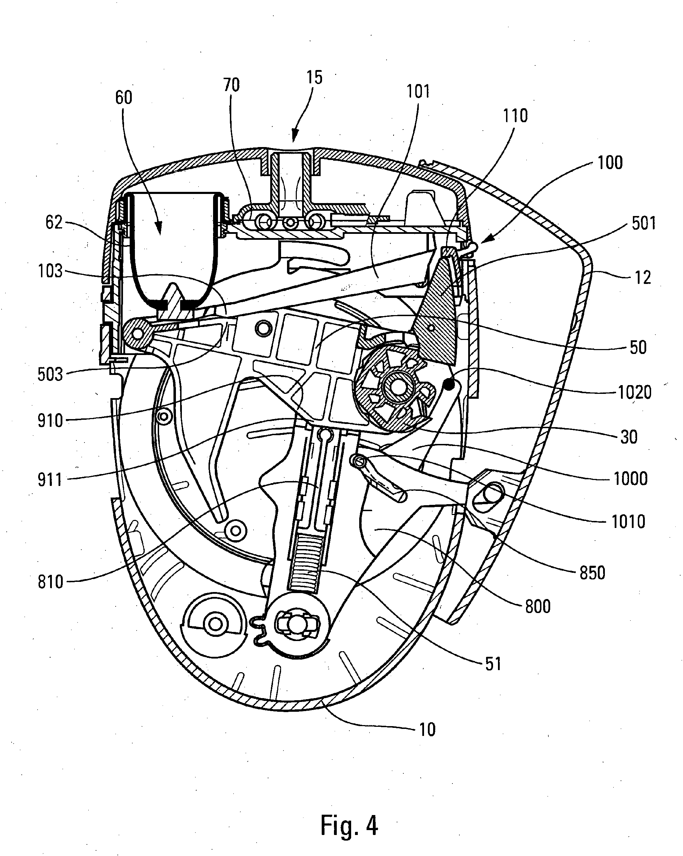

L'élément d'entraînement 1000 est avantageusement monté pivotant à l'intérieur du corps 10. Comme expliqué précédemment, cet élément d'entraînement 1000 coopère d'une part avec la rainure 850 de l'organe de chargement 800 par l'intermédiaire de sa projection 1010. Par ailleurs, une autre projection 1020 dudit élément d'entraînement 1000 coopère avec une denture 37 de la roue de guidage 30. Lorsque l'élément de capot 12 est en position fermée, la projection 1020 est engrenée à l'intérieur de ladite denture 37. Au moment de l'ouverture de l'élément de capot 12, la projection 1010 de l'élément d'entraînement coulisse dans la rainure 850 de l'organe de chargement provoquant le pivotement dudit élément d'entraînement 1000 autour de son axe de pivotement. Ce pivotement fait sortir la projection d'entraînement 1020 de ladite denture 37 de la roue de guidage 30. Cette position désengagée est représentée sur la figure 4. Lorsque l'utilisateur inhale, les moyens de support mobiles 50 se déplacent vers la position de distribution. La roue de guidage 30 étant fixée de manière rotative sur lesdits moyens de support mobiles 50, elle se déplace évidemment ensemble avec ceux-ci en direction des moyens d'ouverture. Dans la position de distribution, la projection d'entraînement 1020 de l'élément d'entraînement 1000 se trouve donc face à une autre dent de la denture 37 de la roue de guidage 30, comme cela est clairement visible sur la figure 5. Lorsque l'utilisateur referme ensuite l'élément de capot 12, la projection 1010 de l'élément d'entraînement coulisse à nouveau dans l'autre sens dans la rainure 850 de l'organe de chargement 800, provoquant le pivotement en retour dudit élément d'entraînement 1000. Ainsi, la projection d'entraînement 1020 vient s'engrener dans l'autre dent, notamment la prochaine, de la denture 37 de la roue de guidage 30, comme cela apparaît sur les figures 6 à 8. Cet engrènement se produit avantageusement au début, notamment dans la première moitié, de la course de retour de l'élément de capot mobile 12 vers sa position de fermeture, et la poursuite de la course de retour provoque la rotation de la roue de guidage 30 sous l'effet dudit élément d'entraînement 1000. De cette manière, la roue de guidage 30 tourne autour de son axe de rotation pour amener le prochain blister plein en face des moyens d'ouverture en vue du prochain actionnement du dispositif et donc de la prochaine distribution d'une dose. Comme expliqué précédemment, la rainure 850 de l'organe de chargement comporte avantageusement deux parties de pente différentes. En partant de la position fermée de l'élément de capot mobile, la première partie de la rainure 850 ne provoque avantageusement pas de pivotement de l'élément d'entraînement 1000 et ce n'est que dans la deuxième partie de pente supérieure de la rainure 850 que l'élément d'entraînement 1000 est amené à pivoter autour de son axe de pivotement pour sortir de la denture 37 de l'élément d'entraînement. Par conséquent, lorsque l'utilisateur referme le capot, la projection 1020 d'entraînement de l'élément d'entraînement 1000 revient rapidement à l'intérieur de la prochaine dent de la denture 37, et la poursuite de la course de retour de l'élément de capot mobile vers sa position de fermeture provoque la rotation de ladite roue de guidage 30.

La surface de came 910 comporte également au moins deux parties de pente différentes, avantageusement séparées par un sommet 911. En partant à nouveau de la position fermée de l'élément de capot mobile, la première partie de pente sur laquelle coulisse la tige 810 permet la compression du ressort 51 comme décrit précédemment. Lorsque le ressort est chargé, c'est-à-dire comprimé, la surface de came 910 prévoit une seconde partie de pente différente avec laquelle la tige 810 va coopérer en position d'ouverture du dispositif. De préférence, la tige 810 exerce une force sensiblement perpendiculaire sur cette seconde partie de surface de came, comme visible sur la figure 4. De cette manière, la position chargée est stable.

En position d'ouverture, représentée sur la figure 4, les moyens de support mobiles 50 qui sont sollicités vers la position de distribution par le ressort comprimé 51 exercent donc une force sur les moyens de blocage 100, en particulier sur l'élément de blocage 110 de ces moyens de blocage par l'intermédiaire de l'extension 501. Avantageusement, du côté opposé de la tige 101, à proximité de la connexion de ladite tige 101 au diaphragme 62, il est prévu une zone d'appui 103 adaptée à coopérer avec une zone complémentaire 503 prévue sur les moyens de support mobiles 50. Cette zone d'appui 103 permet de créer une position chargée stable entre lesdits moyens de support mobiles 50 et lesdits moyens de blocage 100. En effet, ces deux moyens sont chacun mobiles et le double contact, avec d'une part une force exercée vers le haut (en se référant à la position représentée sur la figure 4) par l'extension 501 sur la partie d'épaulement 110, et d'autre part une force exercée vers le bas par la zone d'appui 103 sur la zone complémentaire 503, garantit un équilibre de ce blocage qui ne peut être libéré que par l'inhalation de l'utilisateur provoquant la déformation du diaphragme 62 et donc le pivotement de la tige 101 des moyens de blocage 100.

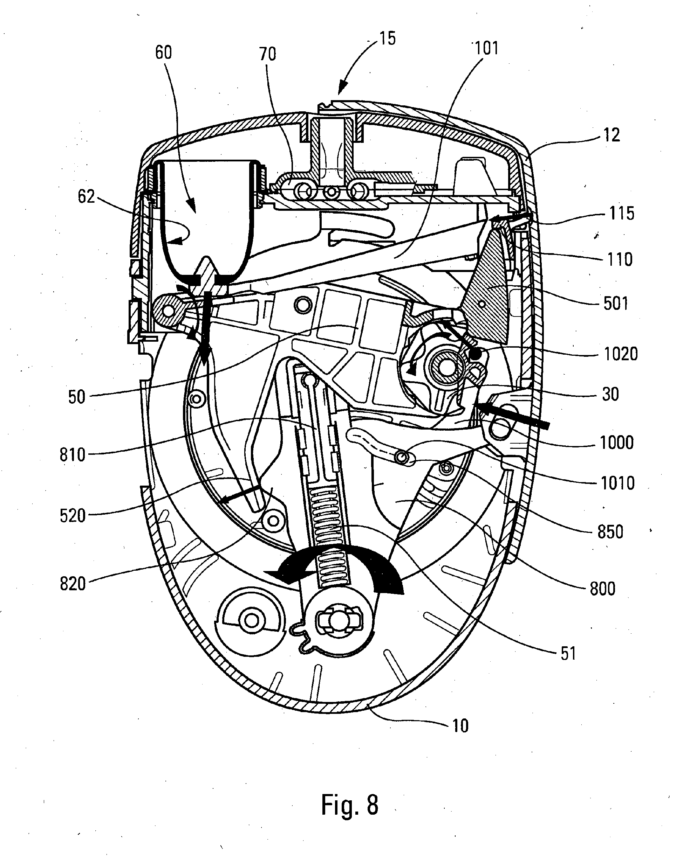

Après l'inhalation, c'est-à-dire en position de distribution représentée sur la figure 5, les moyens de blocage 100 ont pivoté et les moyens de support mobiles 50 ont été déplacés vers le haut par le ressort comprimé 51. Le pivotement des moyens de blocage, en particulier de l'élément de blocage 110, provoque la sortie hors du corps 10 d'une partie d'extrémité 115 de cet élément de blocage 110, comme visible notamment sur la figure 5. Lorsque l'utilisateur referme ensuite l'élément de capot mobile 1.2, ledit élément de capot 12 vient en fin de fermeture pousser sur ladite partie d'extrémité 115 ce qui ramène les moyens de blocage 100 vers leur position initiale avec le diaphragme 62 qui est également ramené dans sa position initiale, comme visible notamment sur la figure 8.

Ainsi, lorsque l'utilisateur ouvre l'inhalateur il charge le système. S'il n'inhale pas et qu'il referme l'inhalateur, celui-ci reprend simplement sa position de départ sans déplacer les réservoirs 21 ni les moyens de blocage 100. Il n'y a donc aucun risque qu'un réservoir (et donc une dose de produit actif) soit perdu par un actionnement malencontreux ou incomplet dans lequel l'utilisateur n'exercerait pas d'inhalation entre l'ouverture et la fermeture. L'ouverture du réservoir, son vidage, la distribution de la poudre dans les poumons de l'utilisateur ainsi que le déplacement de la bande de blisters pour amener un nouveau réservoir plein en face des moyens d'ouverture n'est donc possible que si l'utilisateur inhale.

Par ailleurs, après inhalation et donc déplacement des moyens de support mobiles 50 vers la position de distribution, la fermeture de l'élément de capot mobile 12 ramène l'organe de chargement 800 vers sa position de départ. Comme visible sur la figure 6, c'est à ce moment là que la projection 820 de l'organe de chargement 800 coopère avec la partie d'extension 520 des moyens de support mobiles 50 pour pousser lesdits moyens de support mobiles 50 et ainsi les faire pivoter vers leur position de non-distribution. Il y a donc avantageusement un retour des moyens de support mobiles 50 vers la position de non-distribution qui est mécaniquement liée à la fermeture de l'élément de capot mobile 12.

Comme expliqué précédemment, les figures 2 à 9 ne représentent qu'un seul élément de capot mobile 12, mais il est entendu qu'un second élément de capot mobile, avantageusement symétrique par rapport à celui représenté, pourrait être prévu autour du corps 10, comme cela apparaît sur la figure 1. Avantageusement, ces deux éléments de capot mobiles 11, 12 sont alors engrenés l'un dans l'autre pour garantir une ouverture et une fermeture symétrique desdits deux éléments de capot mobiles. Cet engrenage peut se faire à proximité de leur point de pivotement.

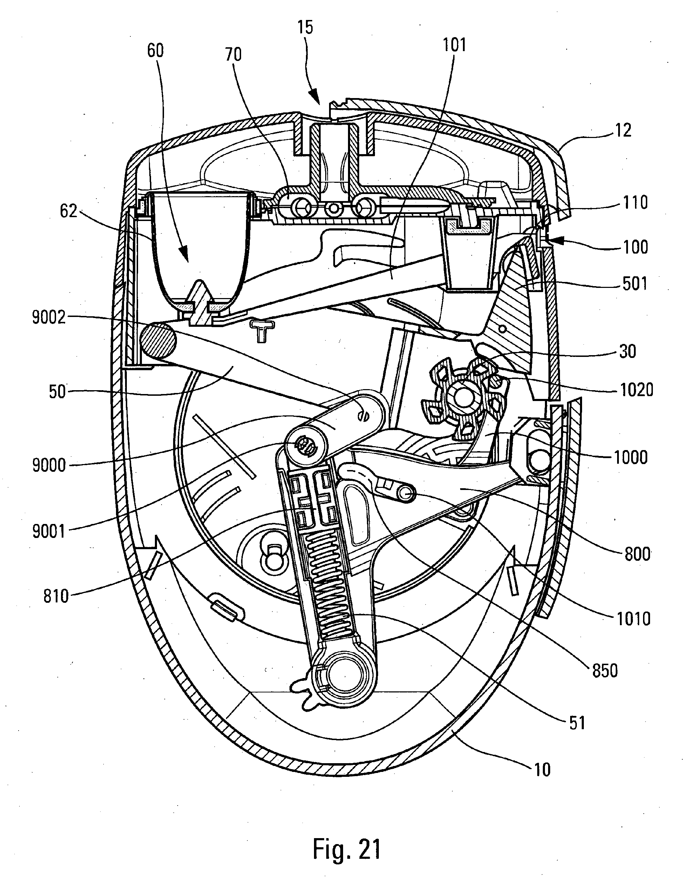

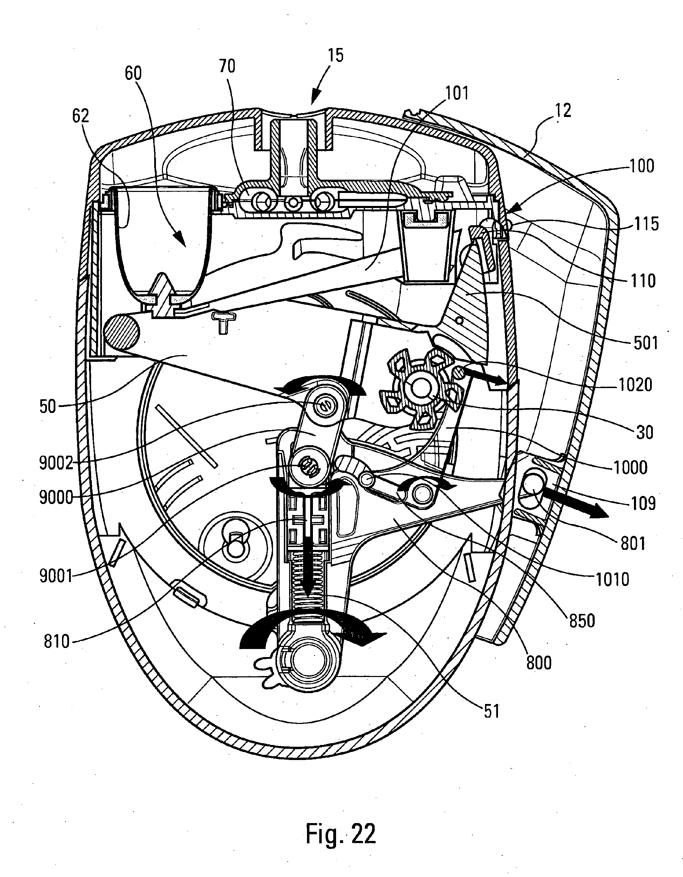

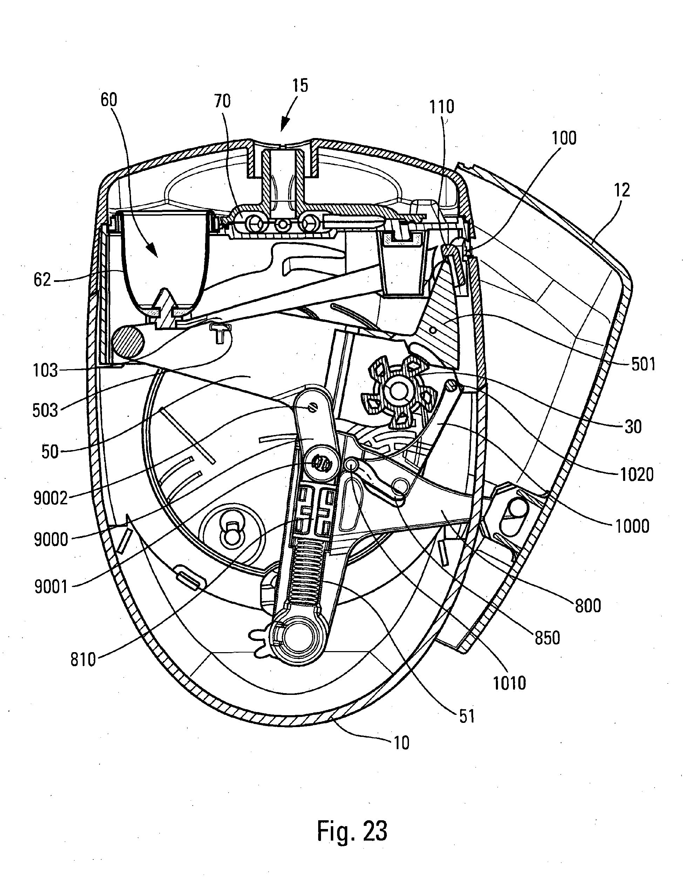

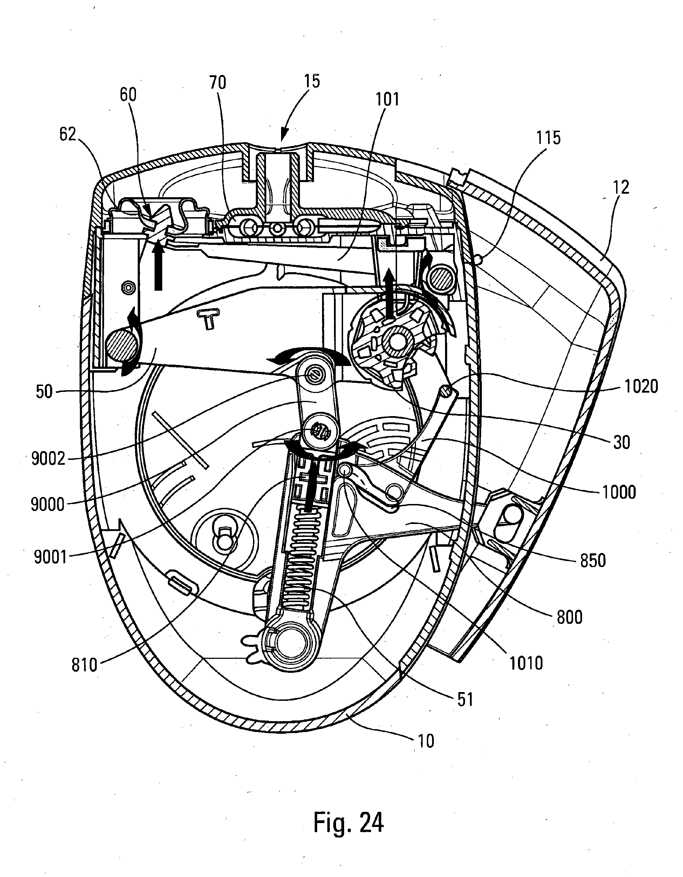

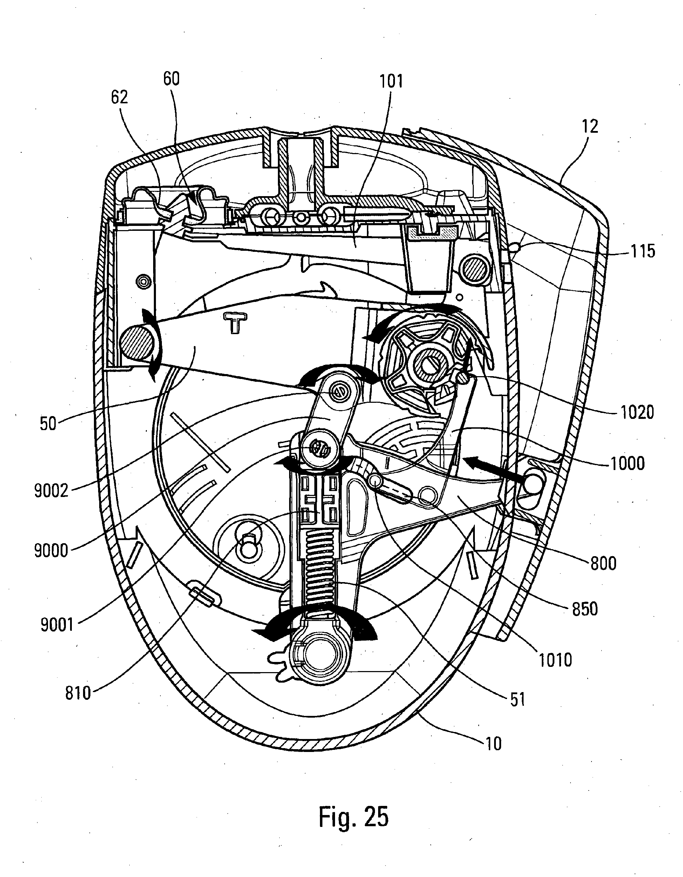

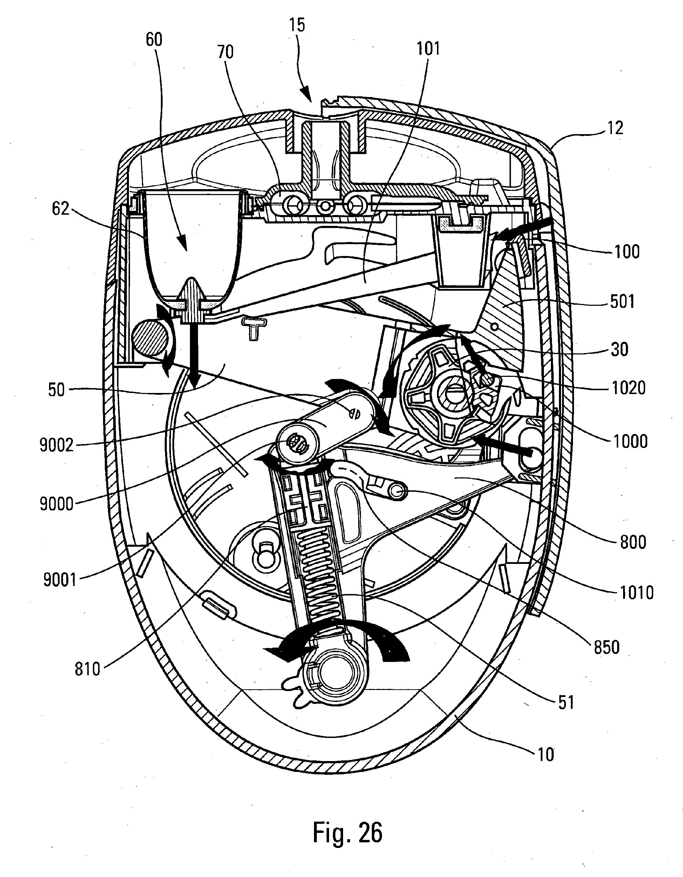

Les figures 21 à 26 montrent une variante de réalisation, dans laquelle la surface de came 910 est remplacée par une bielle ou genouillère 9000 connectée de manière pivotante d'une part à la tige 810 reliée au ressort 51, et d'autre aux moyens de support mobiles 50, par l'intermédiaire de deux axes de pivotement respectif 9001 et 9002. En position de fermeture, représentée sur la figure 21, la genouillère 9000 fait un angle par rapport à la tige 810 et à l'axe longitudinal du ressort 51. Lorsque l'utilisateur ouvre les éléments de capot 11, 12, il tire sur l'axe 9001 en déplaçant l'organe de chargement 800 vers la droite sur la figure, comme indiqué par les flèches sur la figure 22. Les moyens de support mobiles 50 étant fixes, car retenu par les moyens de blocage 100, la genouillère se redresse donc pour s'aligner avec l'axe de la tige 810 et du ressort 51, comme visible sur la figure 22. Ceci provoque la compression du ressort 51 du fait de la forme de la genouillère, plus longue que large. En position d'ouverture, visible sur la figure 23, la genouillère a avantageusement dépassé l'axe longitudinal de la tige 810 et du ressort 51, pour assurer une position d'ouverture stable. Comme précédemment, si l'utilisateur referme l'inhalateur sans inhaler, il ne se passe rien au niveau de la bande de blister, qui n'a pas bougé. Après inhalation (figure 24), les moyens de blocage se libèrent, comme décrit précédemment, un réservoir est ouvert et une dose est expulsée. Ensuite, lorsque l'utilisateur referme les éléments de capot 11,12 (figures 25 et 26), la genouillère 9000 pivote en retour vers sa position initiale, ce qui ramène les moyens de support mobiles 50 dans leur position de non distribution. Les moyens de blocage 100 et les moyens de solidarisation 1000 sont sensiblement identiques à ceux du mode de réalisation des figures 2 à 9, mais des variantes sont aussi envisageables.

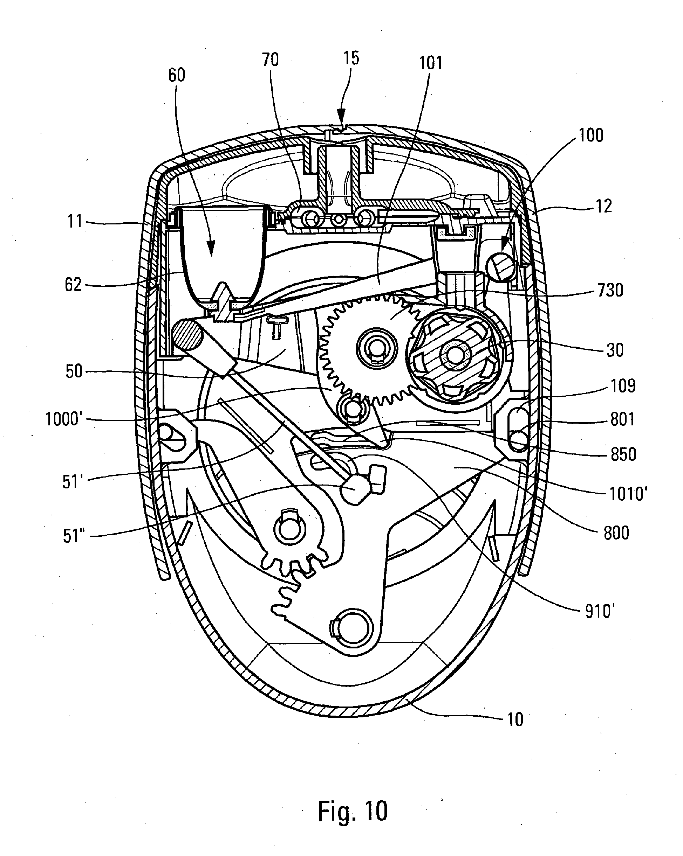

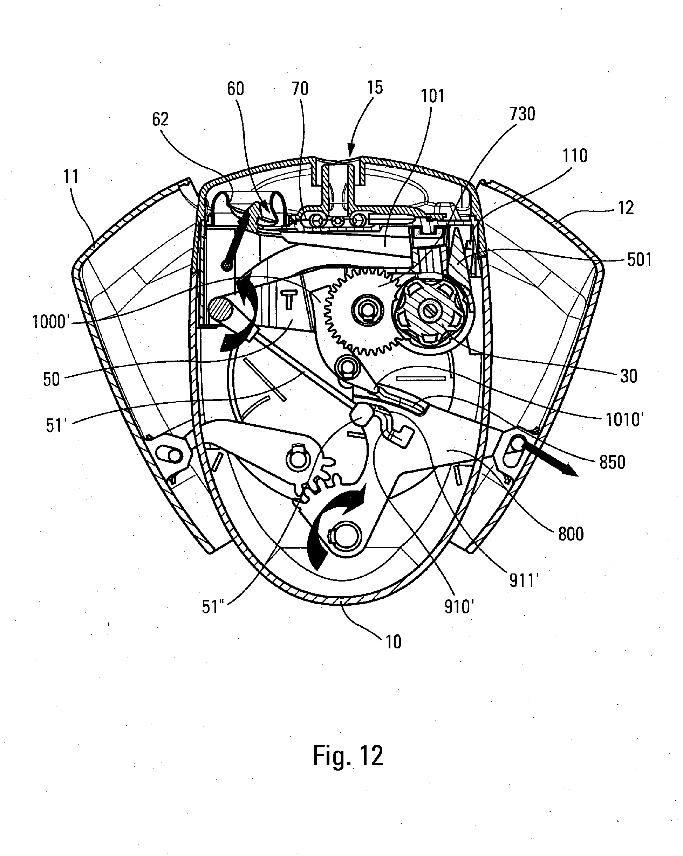

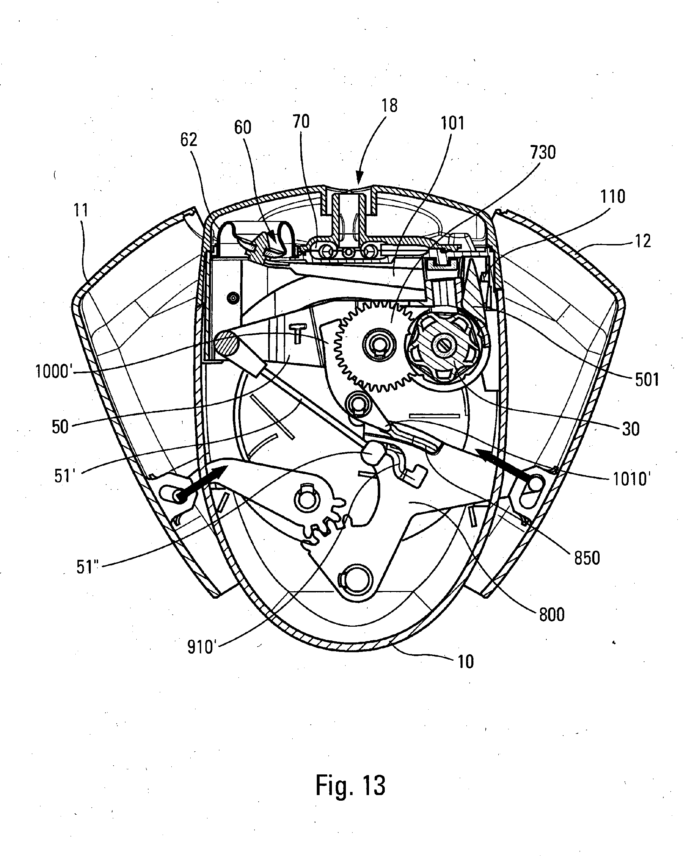

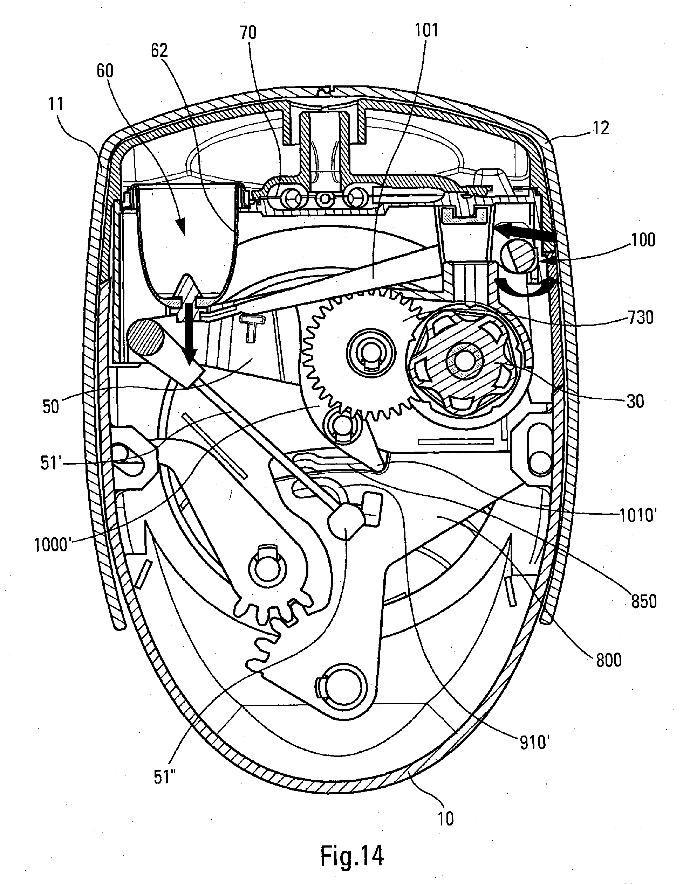

Les figures 10 à 14 représentent un autre mode de réalisation de l'invention. Dans ce mode de réalisation, les éléments identiques ou similaires seront représentés par des références numériques identiques, alors que les éléments qui diffèrent sont représentés par des références numériques comportant des primes.

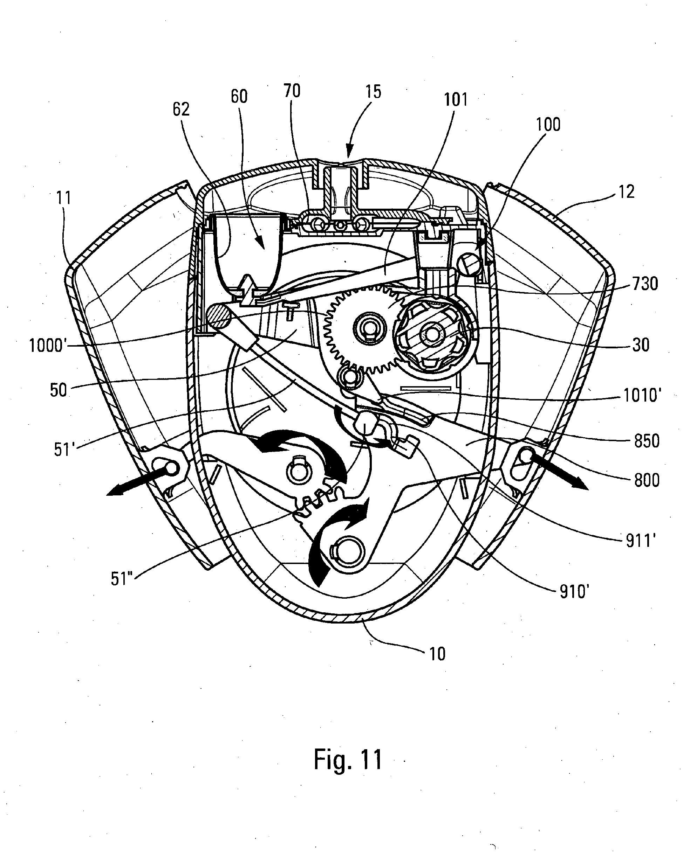

Ce mode de réalisation diffère principalement des précédents par les moyens de chargement qui sont réalisés différemment. Dans ce second mode de réalisation, il n'y a plus de ressort 51 mais une tige 51' qui peut se fléchir pour exercer la force élastique sur les moyens de support mobiles 50. Cette tige 51' est donc d'une part fixée sur les moyens de support mobiles 50, et d'autre part elle est connectée à l'organe d'armement 800, avantageusement par l'intermédiaire d'une projection 51" pénétrant dans une rainure 910' de forme appropriée. Cette rainure forme une surface de came 910' contre laquelle vient coulisser ladite projection 51" de la tige 51' lors de l'ouverture et de la fermeture des éléments de capot mobiles 11, 12. La forme de cette rainure, environ en forme d'arc de cercle ou de partie arquée, comporte ainsi une première partie de rainure et une seconde partie de rainure reliées au niveau d'un sommet 911'. Comme pour la surface de came 910 du premier mode de réalisation des figures 2 à 9, la première partie de la rainure 910' sert à charger le ressort, c'est-à-dire la tige élastique 51', en la déformant, alors que la seconde partie de la rainure 910' permet d'assurer une position stable en position chargée, c'est-à-dire en position d'ouverture. Lorsque le dispositif est chargé comme représenté sur la figure 11, la tige flexible fléchie 51' exerce donc une force sur les moyens de support mobiles 50 pour les solliciter en direction de leur position de distribution. De manière similaire au premier mode de réalisation des figures 2 à 9, ces moyens de support mobiles 50 sont maintenus en position de non distribution par les moyens de blocage 100, qui peuvent être réalisés de manière très similaire à celle décrite précédemment. Lorsque l'utilisateur inhale, les moyens de blocage 100 sont libérés et les moyens de support mobiles 50 peuvent se déplacer vers leur position de distribution en direction des moyens d'ouverture.

Une autre différence de ce mode de réalisation concerne les moyens de déplacement du support de réservoir, en particulier la bande de blisters. En effet, la roue de guidage 30 est, dans ce second mode de réalisation, engrenée avec une roue dentée 730, elle-même en coopération avec un élément d'entraînement 1000'. Alors que dans le premier mode de réalisation des figures 2 à 9, l'élément d'entraînement 1000 était relié d'une part à la rainure 850 de l'organe de chargement 800 et d'autre part directement à la denture 37 de la roue de guidage 30, ici l'élément d'entraînement 1000' est relié d'une part à la rainure 850 de l'organe de chargement 800, comme dans le premier mode de réalisation, mais d'autre part il est connecté à la roue dentée 730 interposée entre l'élément d'entraînement 1000' et la roue de guidage 30. Pour le reste, le fonctionnement est similaire au premier mode de réalisation, à savoir que lorsque l'utilisateur ouvre les éléments de capot mobiles 11,12, l'élément d'entraînement 1000' coulisse dans la rainure 850, avantageusement au niveau d'une projection 1010'. La première partie de rainure 850 étant sensiblement parallèle ou équidistante par rapport à l'axe de pivotement des éléments de capot mobiles 11,12, cette première partie de rainure ne provoque sensiblement pas d'action sur l'élément d'entraînement 1000', alors que la seconde partie de rainure, de pente différente, provoque un pivotement dudit élément d'entraînement 1000' qui se désengrène de la roue dentée 730. Si l'utilisateur referme le dispositif sans inhaler, l'élément d'entraînement 1000' coulisse simplement en retour dans la rainure 850 et revient s'engrener dans la même dent de la roue dentée 730, de sorte qu'il ne se passe rien pour le support de réservoir ou pour les moyens de blocage 100. Par contre, après inhalation, lorsque l'utilisateur referme les éléments de capot mobiles, l'élément d'entraînement 1000' vient s'engrener dans une autre dent de la roue dentée, de manière similaire à ce qui a été décrit précédemment avec le premier mode de réalisation.

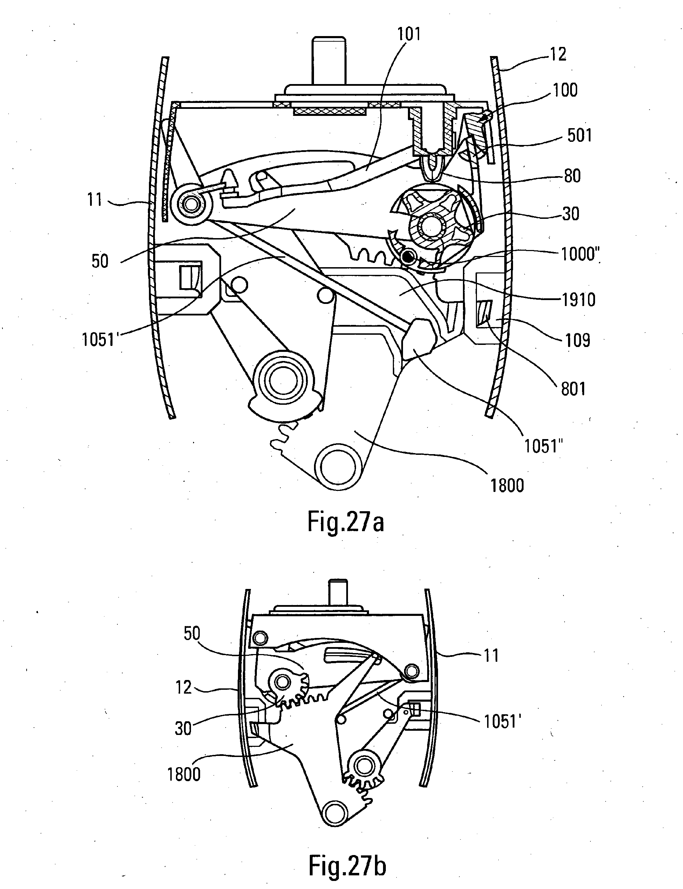

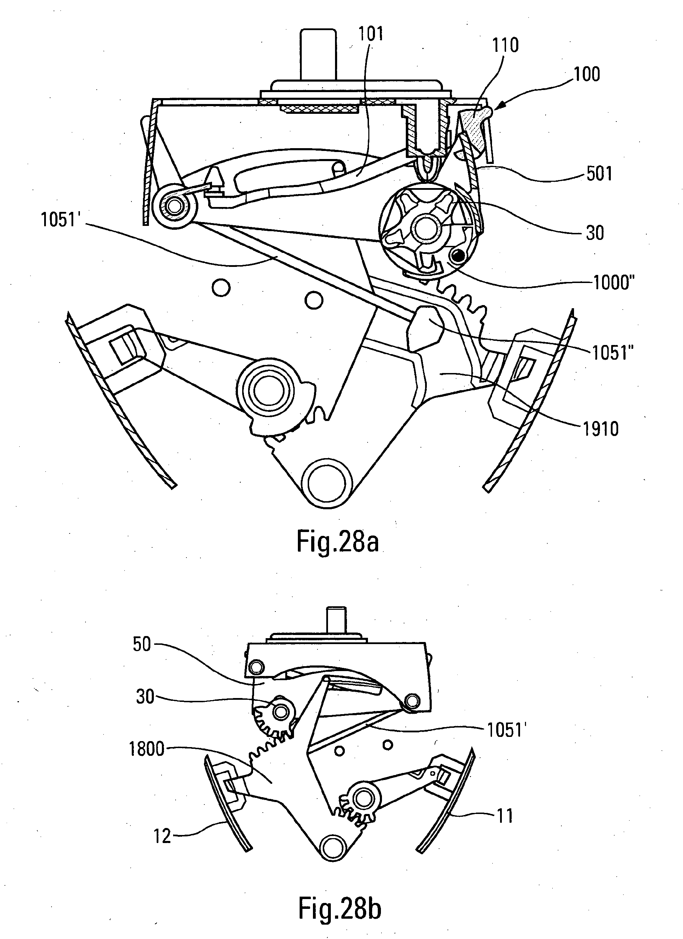

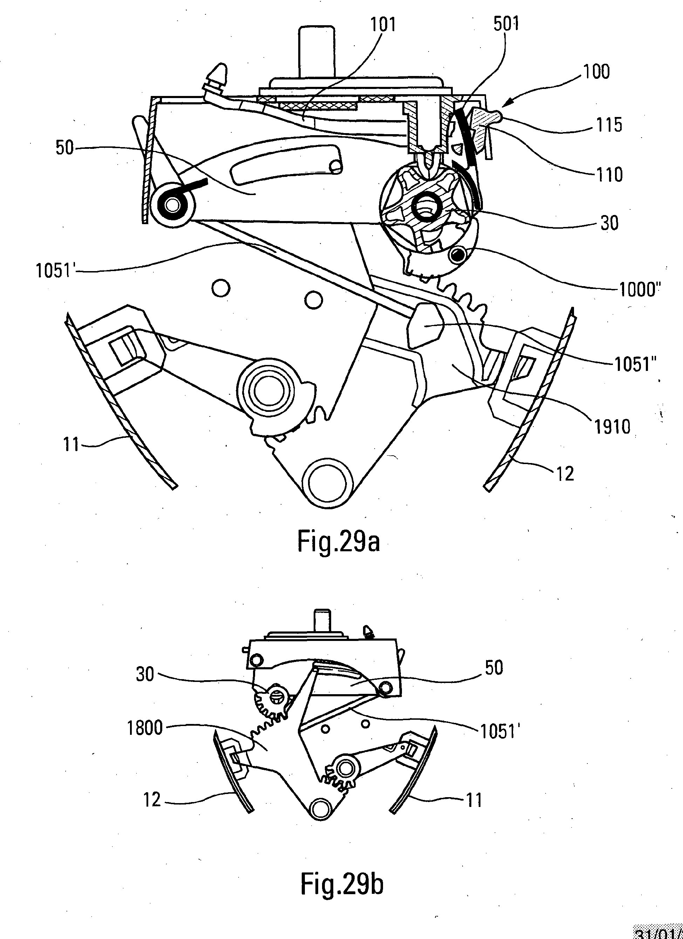

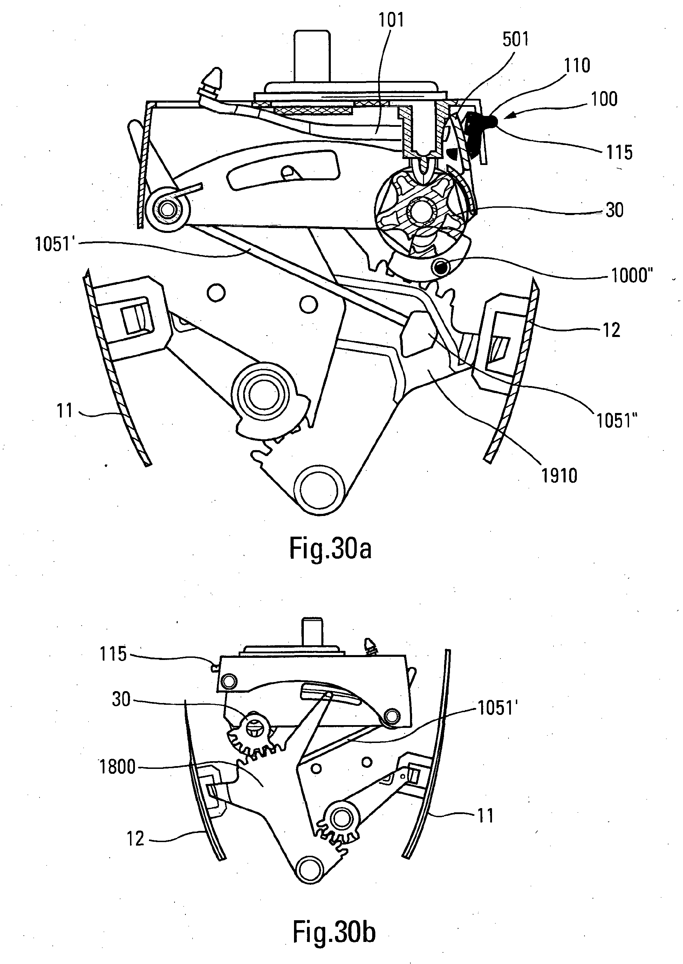

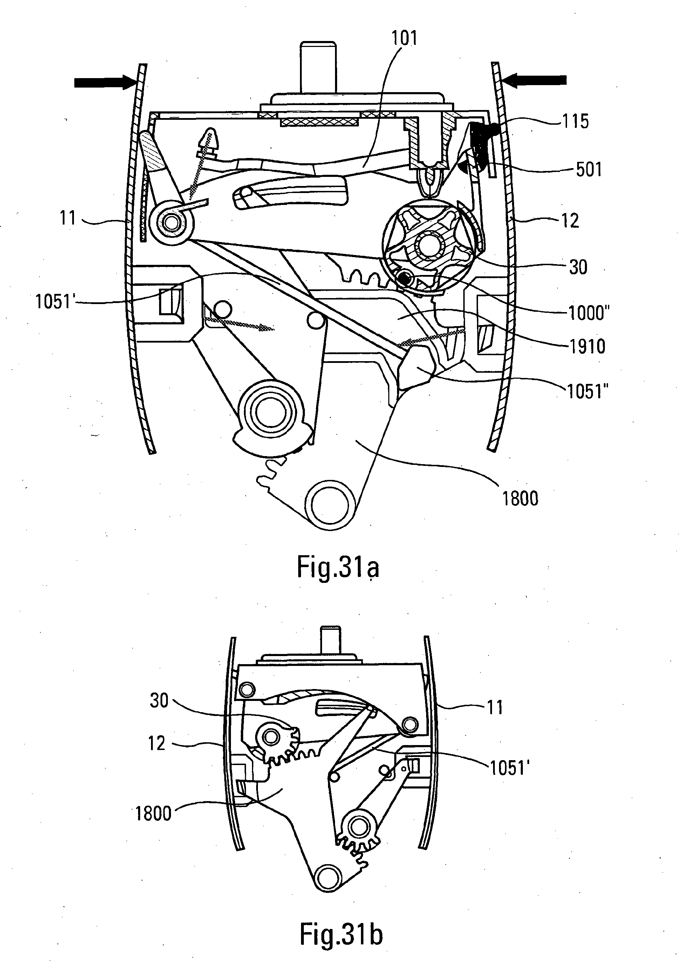

Les figures 27a à 31b montrent un autre mode de réalisation, dans lequel le support de réservoir 20 est déplacé dans la première direction à chaque ouverture des éléments de capot 11, 12. Dans cette variante, si l'utilisateur referme sans inhaler, il ramène le support de réservoir en retour vers sa position initiale. En l'absence d'inhalation, le support de réservoir effectue donc un mouvement de va-et-vient pour se retrouver exactement à sa position de départ après la fermeture. Ceci garantit donc également de ne pas perdre de dose, même en cas de manipulation incomplète du dispositif. En cas d'inhalation, la fermeture après inhalation ne provoque pas de déplacement du support de réservoir, de sorte que pour le prochain actionnement, ce sera le prochain réservoir plein qui sera amené en face des moyens d'ouverture lors de l'ouverture des éléments de capot.

Dans ce mode de réalisation, les moyens de chargement comprennent une tige 1051' fixée d'une part aux moyens de support mobiles 50, et d'autre part coulissant par l'intermédiaire d'une projection 1051" dans une rainure 1910 prévue dans un organe de chargement 1800, relié auxdits éléments de capot mobiles 11, 12. Le fonctionnement de l'armement est similaire à celui décrit en référence aux figures 10 à 14. L'organe de chargement 1800 est engrené avec la roue de guidage 30, comme cela est mieux visible sur les vues de derrière. Des moyens de solidarisation 1000" sont prévus pour coopérer avec la roue de guidage 30 tant que les moyens de support mobiles 50 n'ont pas été déplacés dans leur position de distribution, après une inhalation. Après inhalation, ces moyens de solidarisation, comportant avantageusement une projection coopérant avec une denture de la roue de guidage 30, sont désactivés, c'est-à-dire qu'ils ne coopèrent plus avec la roue de guidage. Lorsque l'utilisateur referme le dispositif, la roue de guidage revient donc ensemble avec les moyens de support mobiles vers la position de non distribution, sans tourner autour de son axe de rotation. Ce n'est qu'en fin de fermeture que les moyens de solidarisation s'engrènent à nouveau avec la roue de guidage.

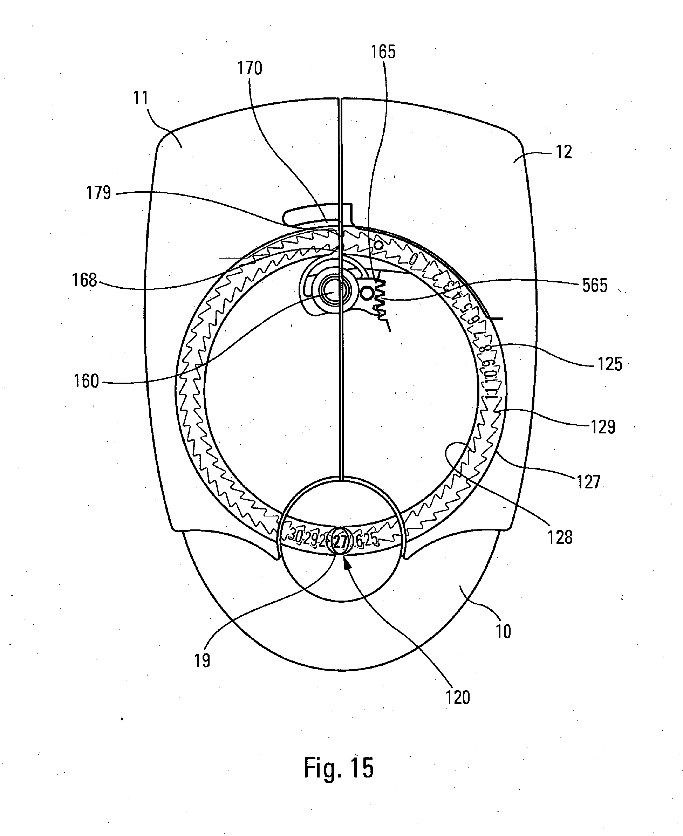

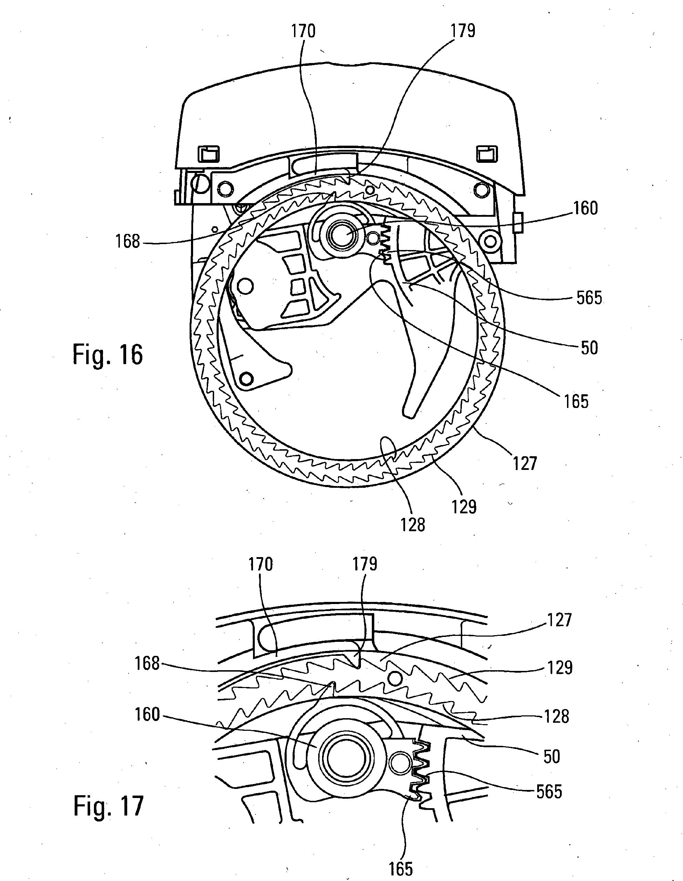

Le dispositif de l'invention peut également comporter une indicateur ou compteur de doses 120, adapté à compter ou à indiquer à l'utilisateur le nombre de doses distribuées ou restant à distribuer. Dans l'exemple représenté, l'indicateur est adapté à compter 60 doses. Les figures 15 à 20 montrent un cycle d'actionnement du dispositif et la manière dans laquelle l'indicateur est actionné. Cet indicateur comporte avantageusement une bague 127 pourvue d'une denture interne 128 et d'une denture externe 129 et comportant des chiffres 125, par exemple de 0 à 60, imprimés sur une de ses faces. La bague est montée de telle sorte que les chiffres passent successivement dans la fenêtre 19 du corps 10. La denture interne 128 est avantageusement adaptée à coopérer avec un actionneur 160, alors que la denture externe 129 est avantageusement adaptée à coopérer avec des moyens anti-retour 170, qui sont adaptés à éviter une rotation de la bague d'indication 127 en sens inverse de celui qui lui est imparti par l'actionneur 160.

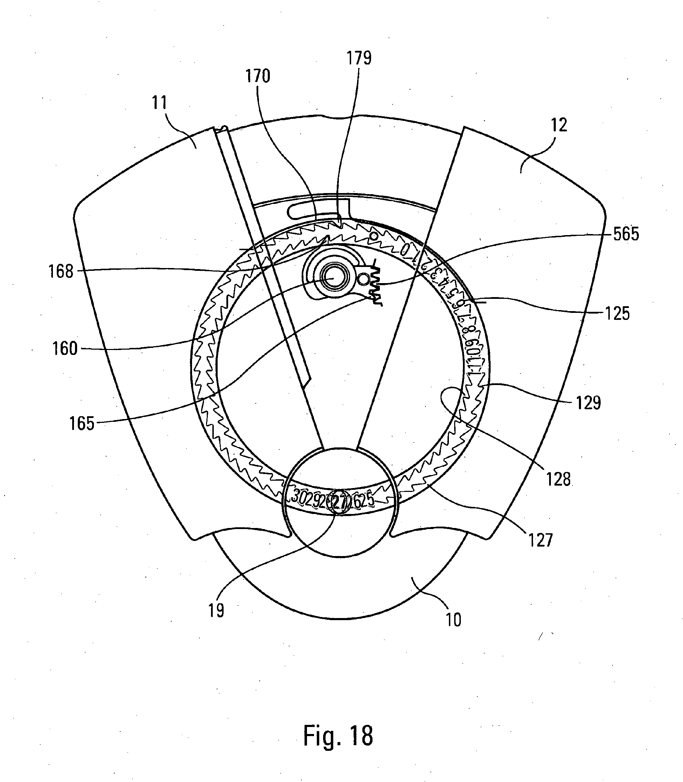

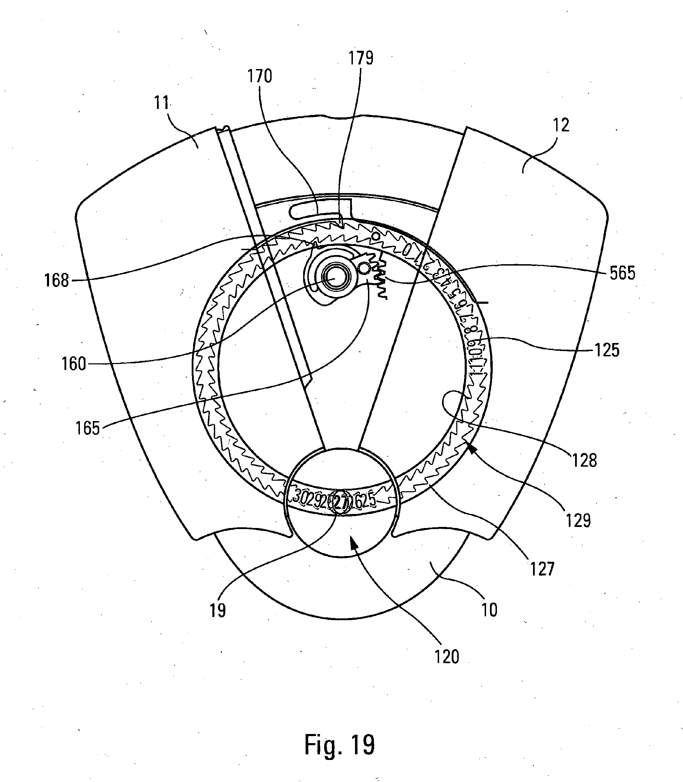

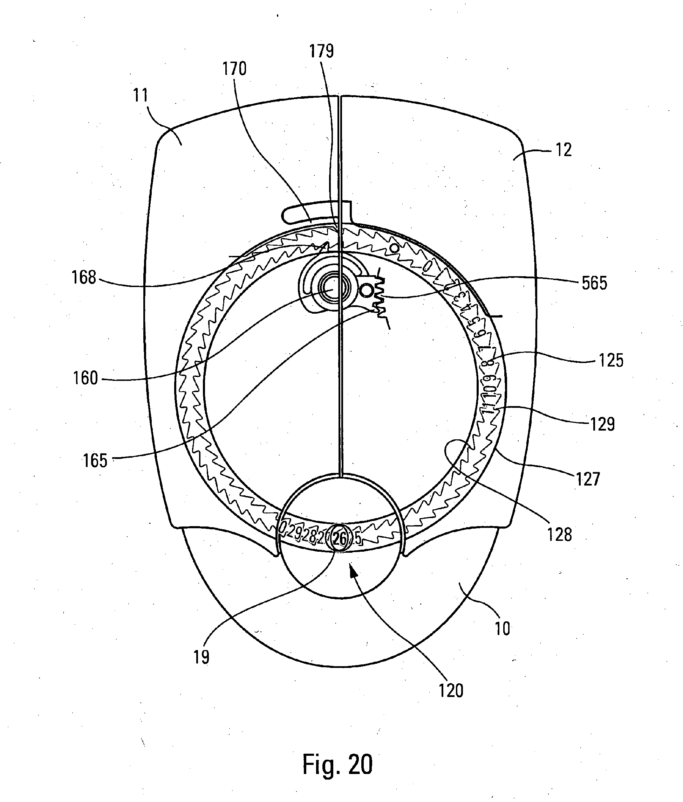

Un but de l'invention est d'éviter de compter des doses qui ne sont pas distribuées, par exemple en cas d'erreur de manipulation, ou d'une manipulation incomplète du dispositif. Il est donc essentiel que le compteur ou indicateur ne soir actionné qu'une fois que l'utilisateur a inhalé, puisque c'est cette inhalation qui conditionne l'ouverture et la distribution de la dose de chaque blister. Pour ce faire, le dispositif comporte un actionneur 160 monté pivotant sur le corps 10. Cet actionneur 160 comporte des moyens de connexion 165, notamment des dents, adaptés à s'engrener dans une denture 565 ou des dents complémentaires prévues sur des moyens de support mobiles 50. Ainsi, lorsque l'utilisateur ouvre le dispositif et qu'il charge les moyens de chargement du dispositif, les moyens de support mobiles 50 ne bougent pas puisqu'ils sont maintenus en position de non-distribution par les moyens de blocage 100. Il ne se passe donc rien au niveau de l'indicateur puisque l'actionneur 160, monté pivotant sur le corps 10 et engrené avec les moyens de support mobiles 50, reste également immobile. Si l'utilisateur referme le dispositif sans inhaler, il ne se passe évidemment rien non plus puisque les moyens de support mobiles 50 restent toujours immobiles. De cette manière, on garantit que l'indicateur ne compte pas de dose s'il n'y a pas d'inhalation. A partir de la position chargée, représentée sur la figure 18, si l'utilisateur inhale, il provoque le déplacement des moyens de support mobiles 50 dans leur position de distribution en direction des moyens d'ouverture. Ce déplacement provoque donc le pivotement de l'actionneur 160 dans une première direction comme visible sur les figures 18 et 19: L'actionneur 160 comporte un doigt 168 engrené dans la denture interne 128 de la bague d'indication 127. Dans cette première direction de déplacement, le doigt 168 de l'actionneur peut glisser sur la pente inclinée de la dent correspondante pour venir se positionner face à la prochaine dent. Parallèlement, les moyens anti-retour 170, en particulier un doigt anti-retour 179, coopèrent avec la denture externe 129 de la bague 127 pour empêcher une rotation de celle-ci sous l'effet du frottement, exercé par exemple par le doigt 168 de l'actionneur sur la denture interne 128. Après l'inhalation, lorsque l'utilisateur referme le dispositif, les moyens de support mobiles 50 sont ramenés vers leur position de repos, c'est-à-dire de non-distribution. Ce mouvement provoque donc un pivotement de l'actionneur 160 en sens inverse du précédent, puisque les engrenages respectifs 165, 565 de l'actionneur et des moyens de support mobiles pivotent en sens inverse à celui décrit précédemment. Dans ce déplacement en sens inverse, le doigt 168 de l'actionneur 160 pousse à l'intérieur de la dent dans laquelle il est positionné pour faire tourner la bague 127, comme visible sur la figure 20. Parallèlement, le doigt anti-retour 179 glisse sur la pente inclinée de la dent pour venir se positionner dans la dent suivante. Dans l'exemple représenté, l'indicateur est adapté à indiquer le nombre de doses restant à distribuer de sorte que le chiffre affiché diminue à chaque actionnement. L'inverse est bien entendu également possible, à savoir un compteur qui compte le nombre de doses distribuées. Avantageusement, on peut prévoir des moyens de blocage de l'indicateur après distribution de la dernière dose. Ces moyens de blocage peuvent prendre différentes formes, une forme avantageuse étant de prévoir une dent de forme différente sur la denture interne de sorte que l'actionneur ne peut plus venir s'engrener dans la dent suivante pour continuer à faire tourner ladite bague d'indication. D'autres moyens pour bloquer la rotation de la bague après distribution de la dernière dose sont également envisageables.

Dans tous les modes de réalisation décrits ci-dessus, la bande de blister est formée par une bande présentant deux extrémités. En variante, on pourrait utiliser une bande continue. D'autres modifications sont également possibles sans sortir du cadre de la présente invention.

La présente invention permet donc de fournir un inhalateur de poudre sèche qui procure notamment les fonctions suivantes :
■ une pluralité de doses individuelles de poudre stockées dans des réservoirs individuels étanches, par exemple 30 ou 60 doses ■ stockées sur une bande enroulée en bobine ;
■ la poudre libérée au moyen d'un perçage actionné par l'inhalation de l'utilisateur, ce perçage du blister étant réalisé au moyen d'un système de détection d'inhalation couplé à un système de libération préchargé ;
■ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
■ des moyens pour éviter une perte de dose en cas d'ouverture de l'inhalateur mais en l'absence d'inhalation. Dans ce cas, lors de la fermeture de l'inhalateur, le dispositif revient exactement à sa position de départ ;
■ un indicateur de doses adapté à compter les doses seulement en cas d'inhalation.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

L'inhalateur de l'invention, incorporant toutes ou parties des fonctions décrites, s'avère fournir des performances supérieures à celles des dispositifs existants. En particulier, l'inhalateur de l'invention fournit de préférence un taux de vidage des réservoirs d'au moins 90% à chaque actionnement. Avantageusement, ce taux de vidage, correspondant au pourcentage du produit fluide expulsé hors d'un réservoir ouvert lors d'un actionnement du dispositif, est supérieur à 95%, de préférence même supérieur à 97%. Ce taux de vidage élevé est notamment même supérieur aux performances obtenues avec des inhalateurs actifs qui sont généralement plus efficaces que les inhalateurs passifs, et dans lesquels ce n'est pas l'écoulement d'inhalation qui vide le blister et expulse la dose mais un écoulement d'air comprimé libéré au moment de l'inhalation. Il garantit une efficacité maximale du dispositif de l'invention. Couplé à l'ouverture déclenchée par l'inhalation, ce taux de vidage élevé garantit une distribution optimale du produit fluide, en l'occurrence la poudre, dans les poumons de l'utilisateur. Par ailleurs, l'invention fournit également une meilleure régularité de vidage des réservoirs lors d'actionnements successifs. Ainsi, en se référant par exemple à 10 réservoirs d'une bande de blisters, il s'avère que le taux de vidage varie de moins de 15%, avantageusement de moins de 10%, de préférence de moins de 5% d'un réservoir à l'autre. Cette meilleure régularité garantit une meilleure reproductibilité de la dose et donc aussi une meilleure efficacité du dispositif de l'invention.

Diverses modifications sont également possibles pour un homme du métier sans sortir du cadre de la présente invention tel que défini par les revendications annexées. En particulier, les diverses caractéristiques et fonctionnalités du dispositif décrites en référence aux différents modes de réalisation et variantes peuvent être adaptées à tous ces modes de réalisation et variantes, et peuvent être combinées entre elles d'une quelconque façon appropriée.

## Revendications

1. Dispositif de distribution de produit fluide, comportant :
- un corps (10) pourvu d'un orifice de distribution,
- au moins un élément de capot (11, 12) mobile entre une position de fermeture et une position d'ouverture,
- une pluralité de réservoirs individuels (21) contenant chacun une dose de produit fluide, telle que de la poudre pharmaceutique, lesdits réservoirs étant formés sur un support de réservoirs (20),
- des moyens de support mobiles (50) montés pivotants sur le corps autour d'un axe de pivotement (511) et recevant ledit support de réservoir et déplaçables entre une position de non-distribution et une position de distribution,
- des moyens d'ouverture de réservoir (80) pour ouvrir un réservoir respectif à chaque actionnement desdits moyens d'ouverture,
- des moyens de déplacement rotatifs (30) dudit support de réservoir dans une première direction pour amener un réservoir plein en face desdits moyens d'ouverture après chaque utilisation du dispositif, ledit support de réservoir étant en outre déplaçable, ensemble avec les moyens de support mobiles, entre ladite position de non-distribution dans laquelle il ne coopère pas avec lesdits moyens d'ouverture, et ladite position de distribution, dans laquelle lesdits moyens d'ouverture ouvrent un réservoir respectif,
- des moyens de chargement (51 ; 51') pour solliciter lesdits moyens de support mobiles vers ladite position de distribution, lesdits moyens de chargement étant chargés par ledit au moins un élément de capot,
- des moyens de blocage (100) pour maintenir lesdits moyens de support mobiles en position de non-distribution,
- des moyens de déclenchement (60), pour libérer lesdits moyens de blocage et permettre le déplacement desdits moyens de support mobiles, et donc dudit support de réservoir, vers ladite position de distribution, lesdits moyens de déclenchement étant actionnés par l'inhalation de l'utilisateur,
**caractérisé en ce que** le dispositif comprend :
- des moyens de solidarisation (1000 ; 1000'), qui lorsqu'ils sont activés, solidarisent lesdits moyens de déplacement avec ledit au moins un élément de capot pour déplacer ledit support de réservoir dans ladite première direction lorsque ledit au moins un élément de capot est ramené de sa position d'ouverture vers sa position de fermeture,
- lesdits moyens de solidarisation étant activés lorsque lesdits moyens de support mobiles sont déplacés vers ladite position de distribution lors de l'inhalation de l'utilisateur, et étant désactivés en l'absence d'inhalation de telle sorte qu'une ouverture suivie d'une fermeture dudit au moins un capot mobile, sans inhalation avant la fermeture, ne déplace pas ledit support de réservoir dans ladite première direction.

2. Dispositif de distribution de produit fluide comportant :
- un corps (10) pourvu d'un orifice de distribution,
- au moins un élément de capot (11, 12) mobile entre une position de fermeture et une position d'ouverture,
- une pluralité de réservoirs individuels (21) contenant chacun une dose de produit fluide, telle que de la poudre pharmaceutique, lesdits réservoirs étant formés sur un support de réservoirs (20),
- des moyens de support mobiles (50) montés pivotants sur le corps autour d'un axe de pivotement (511) et recevant ledit support de réservoir et déplaçables entre une position de non-distribution et une position de distribution,
- des moyens d'ouverture de réservoir (80) pour ouvrir un réservoir respectif à chaque actionnement desdits moyens d'ouverture,
- des moyens de déplacement rotatifs (30) dudit support de réservoir dans une première direction pour amener un réservoir plein en face desdits moyens d'ouverture avant chaque utilisation du dispositif, ledit support de réservoir étant en outre déplaçable, ensemble avec les moyens de support mobiles, entre ladite position de non-distribution dans laquelle il ne coopère pas avec lesdits moyens d'ouverture, et ladite position de distribution, dans laquelle lesdits moyens d'ouverture ouvrent un réservoir respectif,
- des moyens de chargement (1051) pour solliciter lesdits moyens de support mobiles vers ladite position de distribution, lesdits moyens de chargement étant chargés par ledit au moins un élément de capot,
- des moyens de blocage (100) pour maintenir lesdits moyens de support mobiles en position de non-distribution,
- des moyens de déclenchement (60), pour libérer lesdits moyens de blocage et permettre le déplacement desdits moyens de support mobiles, et donc dudit support de réservoir, vers ladite position de distribution, lesdits moyens de déclenchement étant actionnés par l'inhalation de l'utilisateur,
**caractérisé en ce que** le dispositif comprend :
- des moyens de solidarisation (1000"), qui lorsqu'ils sont activés, solidarisent lesdits moyens de déplacement avec ledit au moins un élément de capot pour déplacer ledit support de réservoir dans ladite première direction lorsque ledit au moins un élément de capot est amené de sa position de fermeture vers sa position d'ouverture,
- lesdits moyens de solidarisation étant désactivés lorsque lesdits moyens de support mobiles sont déplacés vers ladite position de distribution lors de l'inhalation de l'utilisateur, et étant activés en l'absence d'inhalation, de telle sorte qu'une ouverture suivie d'une fermeture dudit au moins un élément de capot mobile, sans inhalation avant la fermeture, déplace ledit support de réservoir dans ladite première direction lors de l'ouverture de l'élément de capot et ramène ledit support de réservoir en sens inverse dans sa position initiale lors de la fermeture de l'élément de capot.

3. Dispositif selon la revendication 1 ou 2, dans lequel lesdits moyens d'ouverture de réservoir sont des moyens de perçage fixes par rapport à l'orifice de distribution.

4. Dispositif selon l'une quelconque des revendications précédentes, dans lequel ledit support de réservoir est un bande de blisters avec les réservoirs formés par des blisters disposés les uns à la suite des autres le long de ladite bande.

5. Dispositif selon l'une quelconque des revendications précédentes, dans lequel ledit support de réservoir coopère avec une roue de guidage (30) comportant des évidements (31) recevant lesdits réservoirs, ladite roue étant déplaçable en rotation pour déplacer ledit support de réservoir dans ladite première direction, pour amener un réservoir plein face aux moyens d'ouverture de réservoir avant et/ou après chaque inhalation et déplaçable en direction desdits moyens d'ouverture de réservoir, ensemble avec lesdits moyens de support mobiles, au moment de l'inhalation par un utilisateur pour réaliser l'ouverture dudit réservoir.

6. Dispositif selon la revendication 5, dans lequel ladite roue de guidage est déplaçable par rapport aux moyens d'ouverture de réservoir entre une position de non-distribution et une position de distribution, dans laquelle lesdits moyens d'ouverture de réservoir pénètrent dans un réservoir, ladite roue de guidage étant sollicitée élastiquement vers la position de distribution et étant retenue dans sa position de non-distribution par des moyens de blocage (100), lesdits moyens de blocage étant reliés à un organe mobile (60) déplacé lors d'une inhalation pour libérer lesdits moyens de blocage.

7. Dispositif selon la revendication 5 ou 6, dans lequel ladite roue de guidage comporte une denture (37) coopérant avec un élément d'entraînement (1000 ; 1000') relié à au moins un élément de capot mobile.

8. Dispositif selon la revendication 7, dans lequel lesdits moyens de solidarisation mettent en prise ladite denture et ledit élément d'entraînement lorsqu'ils sont activés et désengagent ladite première denture dudit élément d'entraînement lorsqu'ils sont désactivés.

9. Dispositif selon l'une quelconque des revendications 5 à 8, dans lequel un élément d'entraînement (1000 ; 1000') est monté pivotant sur le corps (10), ledit élément d'entraînement (1000 ; 1000') comportant une première projection (1020 ; 1020') coopérant avec ladite roue évidée (30) et une seconde projection (1010 ; 1010') coopérant avec un organe de chargement (800), relié audit au moins un élément de capot mobile (11, 12).

10. Dispositif selon la revendication 9, dans lequel ledit organe de chargement (800) est monté pivotant sur le corps (10) et comporte des moyens de connexion (801) reliés audit au moins un élément de capot mobile (11, 12) et des moyens de guidage, tels qu'une rainure (850), recevant et guidant ladite seconde projection (1010) dudit élément d'entraînement (1000).

11. Dispositif selon la revendication 10, dans lequel ladite rainure (850) comporte au moins deux parties de rainure, une première partie et une seconde partie d'inclinaison différente, ledit élément d'entraînement (1000 ; 1000') étant pivoté par rapport audit corps seulement lorsque ladite seconde projection coopère avec ladite seconde partie de rainure.

## Patentansprüche

1. Ausgabevorrichtung für ein fluides Produkt, aufweisend:
- einen mit einer Ausgabeöffnung versehenen Körper (10),
- mindestens ein Abdeckelement (11, 12), das zwischen einer Schließposition und einer Öffnungsposition beweglich ist,
- eine Vielzahl von einzelnen Behältern (21), die jeweils eine Dosis eines fluiden Produktes, wie ein pharmazeutisches Pulver, enthalten, wobei die Behälter auf einem Behälterträger (20) ausgebildet sind,
- bewegliche Trägermittel (50), die an dem Körper um eine Schwenkachse (511) verschwenkbar angebracht sind und den Behälterträger aufnehmen und zwischen einer Position, in der keine Ausgabe stattfindet, und einer Ausgabeposition verschiebbar sind,
- Behälteröffnungsmittel (80) zum Öffnen eines jeweiligen Behälters bei jeder Betätigung der Öffnungsmittel,
- drehbare Verschiebemittel (30) des Behälterträgers in eine erste Richtung, um einen vollen Behälter nach jeder Verwendung der Vorrichtung in eine Lage gegenüber den Öffnungsmitteln zu überführen, wobei der Behälterträger des Weiteren zusammen mit den beweglichen Trägermitteln zwischen der Position, in der keine Ausgabe stattfindet und in welcher er nicht mit den Öffnungsmitteln zusammenwirkt, und der Ausgabeposition, in welcher die Öffnungsmittel einen jeweiligen Behälter öffnen, verschiebbar ist,
- Belastungsmittel (51; 51') zum Beanspruchen der beweglichen Trägermittel in Richtung zur Ausgabeposition, wobei die Belastungsmittel durch das mindestens eine Abdeckelement belastet werden,
- Sperrmittel (100) zum Halten der beweglichen Trägermittel in einer Position, in der keine Ausgabe stattfindet,
- Lösemittel (60) zum Freigeben der Sperrmittel und Ermöglichen der Verschiebung der beweglichen Trägermittel und somit des Behälterträgers in die Ausgabeposition, wobei die Lösemittel durch Inhalation durch den Benutzer betätigt werden,
**dadurch gekennzeichnet, dass** die Vorrichtung aufweist:
- Verbindungsmittel (1000; 1000'), die, wenn sie aktiviert sind, die Verschiebemittel mit dem mindestens einen Abdeckelement verbinden, um den Behälterträger in die erste Richtung zu verschieben, wenn das mindestens eine Abdeckelement von seiner Öffnungsposition in seine Schließposition überführt wird,
- wobei die Verbindungsmittel aktiviert sind, wenn die beweglichen Trägermittel bei Inhalation durch den Benutzer in die Ausgabeposition verschoben werden, und deaktiviert sind, wenn keine Inhalation stattfindet, so dass eine Öffnung gefolgt von einer Schließung der mindestens einen beweglichen Abdeckung ohne Inhalation vor der Schließung den Behälterträger nicht in die erste Richtung verschiebt.

2. Ausgabevorrichtung für ein fluides Produkt, aufweisend:
- einen mit einer Ausgabeöffnung versehenen Körper (10),
- mindestens ein Abdeckelement (11, 12), das zwischen einer Schließposition und einer Öffnungsposition beweglich ist,
- eine Vielzahl von einzelnen Behältern (21), die jeweils eine Dosis eines fluiden Produktes, wie ein pharmazeutisches Pulver, enthalten, wobei die Behälter auf einem Behälterträger (20) ausgebildet sind,
- bewegliche Trägermittel (50), die auf dem Körper um eine Schwenkachse (511) verschwenkbar angebracht sind und den Behälterträger aufnehmen und zwischen einer Position, in der keine Ausgabe stattfindet, und einer Ausgabeposition verschiebbar sind,
- Behälteröffnungsmittel (80) zum Öffnen eines jeweiligen Behälters bei jeder Betätigung der Öffnungsmittel,
- drehbare Verschiebemittel (30) des Behälterträgers in eine erste Richtung, um einen vollen Behälter vor jeder Verwendung der Vorrichtung in eine Lage gegenüber den Öffnungsmitteln zu überführen, wobei der Behälterträger des Weiteren zusammen mit den beweglichen Trägermitteln zwischen der Position, in der keine Ausgabe stattfindet und in welcher er nicht mit den Öffnungsmitteln zusammenwirkt, und der Ausgabeposition verschiebbar ist, in welcher die Öffnungsmittel einen jeweiligen Behälter öffnen,
- Belastungsmittel (1051) zum Beanspruchen der beweglichen Trägermittel in Richtung zur Ausgabeposition, wobei die Belastungsmittel durch das mindestens eine Abdeckelement belastet werden,
- Sperrmittel (100) zum Halten der beweglichen Trägermittel in einer Position, in der keine Ausgabe stattfindet,
- Lösemittel (60) zum Freigeben der Sperrmittel und Ermöglichen der Verschiebung der beweglichen Trägermittel und somit des Behälterträgers in die Ausgabeposition, wobei die Lösemittel durch Inhalation durch den Benutzer betätigt werden,
**dadurch gekennzeichnet, dass** die Vorrichtung aufweist:
- Verbindungsmittel (1000"), die, wenn sie aktiviert sind, die Verschiebemittel mit dem mindestens einen Abdeckelement verbinden, um den Behälterträger in die erste Richtung zu verschieben, wenn das mindestens eine Abdeckelement von seiner Schließposition in seine Öffnungsposition überführt wird,
- wobei die Verbindungsmittel deaktiviert sind, wenn die beweglichen Trägermittel bei Inhalation durch den Benutzer in die Ausgabeposition verschoben werden, und aktiviert sind, wenn keine Inhalation stattfindet, so dass eine Öffnung, die von einer Schließung des mindestens einen Abdeckelements gefolgt ist, ohne Inhalation vor der Schließung den Behälterträger bei Öffnung des Abdeckelements in die erste Richtung verschiebt und den Behälterträger bei Schließung des Abdeckelements in die umgekehrte Richtung in seine Ausgangsposition überführt.

3. Vorrichtung nach Anspruch 1 oder 2, wobei die Behälteröffnungsmittel Perforierungsmittel sind, die gegenüber der Ausgabeöffnung befestigt sind.

4. Vorrichtung nach einem der vorhergehenden Ansprüche, wobei der Behälterträger ein Blisterstreifen mit Behältern ist, die durch Blister gebildet sind, die aufeinanderfolgend entlang des Streifens angeordnet sind.

5. Vorrichtung nach einem der vorhergehenden Ansprüche, wobei der Behälterträger mit einem Führungsrad (30) zusammenwirkt, das Vertiefungen (31) umfasst, die die Behälter aufnehmen, wobei das Rad verdrehbar ist, um den Behälterträger in die erste Richtung zu verschieben, um einen vollen Behälter vor und/oder nach jeder Inhalation in eine Lage gegenüber den Behälteröffnungsmitteln zu überführen, und in Richtung der Behälteröffnungsmittel zusammen mit den beweglichen Trägermitteln im Augenblick der Inhalation durch einen Benutzer verschiebbar ist, um die Öffnung des Behälters zu bewirken.

6. Vorrichtung nach Anspruch 5, wobei das Führungsrad in Bezug auf die Behälteröffnungsmittel zwischen einer Position, in der keine Ausgabe stattfindet, und einer Ausgabeposition verschiebbar ist, wobei die Behälteröffnungsmittel in einen Behälter eindringen, wobei das Führungsrad elastisch gegen die Ausgabeposition vorgespannt und durch Sperrmittel (100) in seiner Position, in der keine Ausgabe stattfindet, gehalten wird, wobei die Sperrmittel mit einer beweglichen Einrichtung (60) verbunden sind, die bei einer Inhalation verschoben wird, um die Sperrmittel zu lösen.

7. Vorrichtung nach Anspruch 5 oder 6, wobei das Führungsrad eine Verzahnung (37) aufweist, die mit einem Mitnahmeelement (1000; 1000') zusammenwirkt, das mit mindestens einem beweglichen Abdeckelement verbunden ist.

8. Vorrichtung nach Anspruch 7, wobei die Verbindungsmittel die Verzahnung und das Mitnahmeelement ineinandergreifen lassen, wenn sie aktiviert sind, und die erste Verzahnung mit dem Mitnahmeelement außer Eingriff bringen, wenn sie deaktiviert sind.

9. Vorrichtung nach einem der Ansprüche 5 bis 8, wobei ein Mitnahmeelement (1000; 1000') verschwenkbar auf dem Körper (10) angebracht ist, wobei das Mitnahmeelement (1000; 1000') einen ersten Vorsprung (1020; 1020'), der mit dem ausgesparten Rad (30) zusammenwirkt, und einen zweiten Vorsprung (1010; 1010') aufweist, der mit einer Belastungseinrichtung (800) zusammenwirkt, die mit dem mindestens einen beweglichen Abdeckelement (11, 12) verbunden ist.

10. Vorrichtung nach Anspruch 9, wobei die Belastungseinrichtung (800) verschwenkbar auf dem Körper (10) angebracht ist und Anschlussmittel (801), die mit dem mindestens einen beweglichen Abdeckelement (11, 12) verbunden sind, und Führungsmittel, wie eine Nut (850), aufweist, die den zweiten Vorsprung (1010) des Mitnahmeelements (1000) aufnehmen und führen.

11. Vorrichtung nach Anspruch 10, wobei die Nut (850) mindestens zwei Nutenteile aufweist, und zwar einen ersten Teil und einen zweiten Teil von unterschiedlicher Neigung, wobei das Mitnahmeelement (1000; 1000') in Bezug auf den Körper nur verschwenkbar ist, wenn der zweite Vorsprung mit dem zweiten Nutenteil zusammenwirkt.

## Claims

1. A fluid dispenser device comprising:
· a body (10) provided with a dispenser orifice;
· at least one cover element (11, 12) that is movable between a closed position and an open position;
· a plurality of individual reservoirs (21) each containing a dose of fluid, such as a pharmaceutical powder, said reservoirs being formed on a reservoir substrate (20);
· movable support means (50) pivotably mounted on said body around a pivoting axis (511) and that receive said reservoir substrate, and that are displaceable between a non-dispensing position and a dispensing position;
· reservoir-opening means (80) for opening a respective reservoir each time said opening means are actuated;
· rotating displacement means (30) for displacing said reservoir substrate in a first direction so as to bring a full reservoir into register with said opening means after each use of the device, said reservoir substrate also being displaceable, together with the movable support means, between said non-dispensing position, in which it does not co-operate with said opening means, and said dispensing position, in which said opening means open a respective reservoir;
· loading means (51; 51') for urging said movable support means towards said dispensing position, said loading means being loaded by said at least one cover element;
· blocking means (100) for retaining said movable support means in the non-dispensing position;
· trigger means (60) for releasing said blocking means and for enabling said movable support means, and thus said reservoir substrate, to be displaced towards said dispensing position, said trigger means being actuated by the user inhaling;
**characterized in that** the device comprises:
· connection means (1000; 1000') which, when they are activated, connect said displacement means with said at least one cover element, so as to displace said reservoir substrate in said first direction when said at least one cover element is returned from its open position to its closed position; and
· said connection means being activated while said movable support means are being displaced towards said dispensing position while the user is inhaling, and being deactivated in the absence of any inhalation, such that opening said at least one movable cover and then closing it without inhaling does not displace said reservoir substrate in said first direction.

2. A fluid dispenser device comprising:
· a body (10) provided with a dispenser orifice;
· at least one cover element (11, 12) that is movable between a closed position and an open position;
· a plurality of individual reservoirs (21) each containing a dose of fluid, such as a pharmaceutical powder, said reservoirs being formed on a reservoir substrate (20);
· movable support means (50) pivotably mounted on said body around a pivoting axis (511) and that receive said reservoir substrate, and that are displaceable between a non-dispensing position and a dispensing position;
· reservoir-opening means (80) for opening a respective reservoir each time said opening means are actuated;
· rotating displacement means (30) for displacing said reservoir substrate in a first direction so as to bring a full reservoir into register with said opening means before each use of the device, said reservoir substrate also being displaceable, together with the movable support means, between said non-dispensing position, in which it does not co-operate with said opening means, and said dispensing position, in which said opening means open a respective reservoir;
· loading means (1051) for urging said movable support means towards said dispensing position, said loading means being loaded by said at least one cover element;
· blocking means (100) for retaining said movable support means in the non-dispensing position;
· trigger means (60) for releasing said blocking means and for enabling said movable support means, and thus said reservoir substrate, to be displaced towards said dispensing position, said trigger means being actuated by the user inhaling;
**Characterized in that** the device comprises:
· connection means (1000") which, when they are activated, connect said displacement means with said at least one cover element, so as to displace said reservoir substrate in said first direction when said at least one cover element is brought from its closed position to its open position; and
· said connection means being deactivated while said movable support means are being displaced towards said dispensing position while the user is inhaling, and being activated in the absence of any inhalation, such that opening said at least one movable cover element and then closing it without inhaling displaces said reservoir substrate in said first direction while the cover element is being opened, and returns said reservoir substrate in the opposite direction into its initial position while the cover element is being closed.

3. A device according to claim 1 or claim 2, in which said reservoir-opening means are perforator means that do not move relative to the dispenser orifice.

4. A device according to any preceding claim, in which said reservoir substrate is a blister strip, with the reservoirs being formed by blisters disposed one behind another along said strip.

5. A device according to any preceding claim, in which said reservoir substrate co-operates with a guide wheel (30) that includes recesses (31) for receiving said reservoirs, said wheel being displaceable in turning so as to displace said reservoir substrate in said first direction in order to bring a full reservoir into register with the reservoir-opening means before and/or after each inhalation, and being displaceable towards said reservoir-opening means, together with said movable support means, when the user inhales, in order to open said reservoir.

6. A device according to claim 5, in which said guide wheel is displaceable relative to the reservoir-opening means between a non-dispensing position and a dispensing position in which said reservoir-opening means penetrate into a reservoir, said guide wheel being resiliently urged towards the dispensing position and being retained in its non-dispensing position by blocking means (100), said blocking means being connected to a movable member (60) that is displaced during inhalation so as to release said blocking means.

7. A device according to claim 5 or claim 6, in which said guide wheel includes a set of teeth (37) that co-operates with a drive element (1000; 1000') that is connected to at least one movable cover element.

8. A device according to claim 7, in which said connection means engage said set of teeth with said drive element when they are activated, and disengage said first set of teeth from said drive element when they are deactivated.

9. A device according to any one of claims 5 to 8, in which a drive element (1000; 1000') is pivotally mounted on the body (10), said drive element (1000; 1000') including a first projection (1020; 1020') that co-operates with said recessed wheel (30), and a second projection (1010; 1010') that co-operates with a loading member (800) that is connected to said at least one movable cover element (11, 12).

10. A device according to claim 9, in which said loading member (800) is pivotally mounted on the body (10) and includes: engagement means (801) connected to said at least one movable cover element (11, 12); and guide means, such as a groove (850), for receiving and guiding said second projection (1010) of said drive element (1000).

11. A device according to claim 10, in which said groove (850) comprises at least two groove portions, a first portion and a second portion of different inclinations, said drive element (1000; 1000') being pivoted relative to said body, only while said second projection is cooperating with said second groove portion.
